# EUROPEAN PATENT APPLICATION

(11) **EP 4 825 977 A1**
(43) Date of publication of application: **21.10.2026**
(21) Application number: 24902974.5
(22) Date of filing: 13.12.2024
(51) Int. Cl.: C12Q 1/68, C12N 15/12

(54) **CAPTURE PROBE, FAMILIAL CHYLOMICRONEMIA SYNDROME DETECTION KIT CONTAINING SAME, AND USE**

(30) Priority: 13.12.2023 CN 202311713738
(71) Applicant: GENZYME CORPORATION, Cambridge, Massachusetts 02141 (US)
(72) Inventor: YOU, Dong, Suzhou, Jiangsu 215028 (CN); LI, Feng, Suzhou, Jiangsu 215028 (CN); WANG, Mingjie, Suzhou, Jiangsu 215028 (CN)
(74) Representative: Zwicker, Jörk
(86) International application number: PCT/CN2024/139229
(87) International publication number: WO 2025/124559

(57) **Abstract**

The present application relates to the field of biomedical detection, and particularly to a capture probe, a familial chylomicronemia syndrome detection kit containing the same, and use thereof. The present application aims to detect FCS-related gene mutations stably and accurately, and to provide a more convenient and accurate FCS detection method for clinical practice. It is of great significance for the clinical diagnosis and treatment of FCS.

## Description

### Technical Field

The present application relates to the field of biomedical detection, and in particular to a capture probe, a familial chylomicronemia syndrome detection kit containing the same, and use thereof.

### Background Art

Familial chylomicronemia syndrome (Familial chylomicronemia syndrome, FCS) is an autosomal recessive genetic disease, also referred to as primary hyperlipoproteinemia type I. FCS is characterized by severe fasting hypertriglyceridemia, secondary to the accumulation of triglyceride (TG)-rich lipoproteins, especially chylomicrons, and requires long-term restriction of the fat content in the diet. The clinical manifestations of FCS are most typically eruptive xanthomas, which are commonly seen on the buttocks, thighs, arms, back, and face, and may also involve the oral mucosa, and other symptoms include upper abdominal pain, which may be caused by tension of the liver and spleen capsules, hepatosplenomegaly, and pancreatitis. This disease is rare and lacks specificity, has potentially serious clinical consequences, and siblings of the patient have a 25% chance of having this disease.

At present, the diagnostic criteria for FCS are mainly derived from European clinical practice and are comprehensive diagnostic criteria, mainly relying on factors such as: triglyceride level, the patient's response to drugs for lowering triglyceride levels, and a medical history of frequent attacks of abdominal pain or pancreatitis. Routine laboratory and clinical examinations cannot precisely identify the cause of the disease, and changes in triglyceride levels are also observed in other diseases and are not changes entirely specific to FCS, and therefore, in clinical practice FCS is often missed in diagnosis. Overall, the current diagnostic methods cannot provide accurate and timely FCS diagnostic results, which is not conducive to timely treatment of FCS, and precise identification and localization of FCS still remain challenging.

### Summary of the Invention

In view of this, one objective of one or more embodiments of the present application is to provide a capture probe, and the probe can be used for accurate detection of FCS-related gene mutations.

In one or more embodiments of the present application, a capture probe is provided, which comprises one or more probe set selected from probe set 1 to probe set 6;
probe set 1 comprises one or more probes selected from probes 1 to 26;
probe set 2 comprises one or more probes selected from probes 27 to 64;
probe set 3 comprises one or more probes selected from probes 65 to 112;
probe set 4 comprises one or more probes selected from probes 113 to 139;
probe set 5 comprises one or more probes selected from probes 140 to 382;
probe set 6 comprises one or more probes selected from probes 383 to 425;
the probes 1 to 425 are constructed for target gene fragments located between different start positions and end positions on the following chromosomes and are complementary to and paired with said target gene fragments:

| Probe number | Chromosome | Start position | End position |
|---|---|---|---|
| Probe 1 | chr11 | 116660063 | 116660183; |
| Probe 2 | chr11 | 116660168 | 116660288; |
| Probe 3 | chr11 | 116660273 | 116660393; |
| Probe 4 | chr11 | 116660378 | 116660498; |
| Probe 5 | chr11 | 116660483 | 116660603; |
| Probe 6 | chr11 | 116660588 | 116660708; |
| Probe 7 | chr11 | 116660693 | 116660813; |
| Probe 8 | chr11 | 116660798 | 116660918; |
| Probe 9 | chr11 | 116660903 | 116661023; |
| Probe 10 | chr11 | 116661008 | 116661128; |
| Probe 11 | chr11 | 116661113 | 116661233; |
| Probe 12 | chr11 | 116661218 | 116661338; |
| Probe 13 | chr11 | 116661323 | 116661443; |
| Probe 14 | chr11 | 116661428 | 116661548; |
| Probe 15 | chr11 | 116661533 | 116661653; |
| Probe 16 | chr11 | 116661638 | 116661758; |
| Probe 17 | chr11 | 116661743 | 116661863; |
| Probe 18 | chr11 | 116662282 | 116662402; |
| Probe 19 | chr11 | 116662387 | 116662507; |
| Probe 20 | chr11 | 116662508 | 116662628; |
| Probe 21 | chr11 | 116663076 | 116663196; |
| Probe 22 | chr11 | 116663181 | 116663301; |
| Probe 23 | chr11 | 116663286 | 116663406; |
| Probe 24 | chr11 | 116663391 | 116663511; |
| Probe 25 | chr11 | 116663496 | 116663616; |
| Probe 26 | chr11 | 116663601 | 116663721; |
| Probe 27 | chr12 | 50497271 | 50497391; |
| Probe 28 | chr12 | 50497376 | 50497496; |
| Probe 29 | chr12 | 50497481 | 50497601; |
| Probe 30 | chr12 | 50497586 | 50497706; |
| Probe 31 | chr12 | 50497691 | 50497811; |
| Probe 32 | chr12 | 50497796 | 50497916; |
| Probe 33 | chr12 | 50498337 | 50498457; |
| Probe 34 | chr12 | 50498442 | 50498562; |
| Probe 35 | chr12 | 50499311 | 50499431; |
| Probe 36 | chr12 | 50499416 | 50499536; |
| Probe 37 | chr12 | 50500051 | 50500171; |
| Probe 38 | chr12 | 50500156 | 50500276; |
| Probe 39 | chr12 | 50500568 | 50500688; |
| Probe 40 | chr12 | 50500673 | 50500793; |
| Probe 41 | chr12 | 50501330 | 50501450; |
| Probe 42 | chr12 | 50501435 | 50501555; |
| Probe 43 | chr12 | 50501540 | 50501660; |
| Probe 44 | chr12 | 50501787 | 50501907; |
| Probe 45 | chr12 | 50501892 | 50502012; |
| Probe 46 | chr12 | 50503186 | 50503306; |
| Probe 47 | chr12 | 50503291 | 50503411; |
| Probe 48 | chr12 | 50503396 | 50503516; |
| Probe 49 | chr12 | 50503501 | 50503621; |
| Probe 50 | chr12 | 50503606 | 50503726; |
| Probe 51 | chr12 | 50503711 | 50503831; |
| Probe 52 | chr12 | 50503816 | 50503936; |
| Probe 53 | chr12 | 50503921 | 50504041; |
| Probe 54 | chr12 | 50504026 | 50504146; |
| Probe 55 | chr12 | 50504131 | 50504251; |
| Probe 56 | chr12 | 50504236 | 50504356; |
| Probe 57 | chr12 | 50504341 | 50504461; |
| Probe 58 | chr12 | 50504446 | 50504566; |
| Probe 59 | chr12 | 50504551 | 50504671; |
| Probe 60 | chr12 | 50504656 | 50504776; |
| Probe 61 | chr12 | 50504761 | 50504881; |
| Probe 62 | chr12 | 50504866 | 50504986; |
| Probe 63 | chr12 | 50504971 | 50505091; |
| Probe 64 | chr12 | 50505076 | 50505196; |
| Probe 65 | chr16 | 903614 | 903734; |
| Probe 66 | chr16 | 903719 | 903839; |
| Probe 67 | chr16 | 903824 | 903944; |
| Probe 68 | chr16 | 903929 | 904049; |
| Probe 69 | chr16 | 904034 | 904154; |
| Probe 70 | chr16 | 904139 | 904259; |
| Probe 71 | chr16 | 904244 | 904364; |
| Probe 72 | chr16 | 904349 | 904469; |
| Probe 73 | chr16 | 904454 | 904574; |
| Probe 74 | chr16 | 904559 | 904679; |
| Probe 75 | chr16 | 904664 | 904784; |
| Probe 76 | chr16 | 905087 | 905207; |
| Probe 77 | chr16 | 905192 | 905312; |
| Probe 78 | chr16 | 918924 | 919044; |
| Probe 79 | chr16 | 919029 | 919149; |
| Probe 80 | chr16 | 919863 | 919983; |
| Probe 81 | chr16 | 919968 | 920088; |
| Probe 82 | chr16 | 920709 | 920829; |
| Probe 83 | chr16 | 920814 | 920934; |
| Probe 84 | chr16 | 921141 | 921261; |
| Probe 85 | chr16 | 921246 | 921366; |
| Probe 86 | chr16 | 929550 | 929670; |
| Probe 87 | chr16 | 929655 | 929775; |
| Probe 88 | chr16 | 942987 | 943107; |
| Probe 89 | chr16 | 960911 | 961031; |
| Probe 90 | chr16 | 961016 | 961136; |
| Probe 91 | chr16 | 981622 | 981742; |
| Probe 92 | chr16 | 981727 | 981847; |
| Probe 93 | chr16 | 983971 | 984091; |
| Probe 94 | chr16 | 984076 | 984196; |
| Probe 95 | chr16 | 984170 | 984290; |
| Probe 96 | chr16 | 1004337 | 1004457; |
| Probe 97 | chr16 | 1004442 | 1004562; |
| Probe 98 | chr16 | 1004547 | 1004667; |
| Probe 99 | chr16 | 1004652 | 1004772; |
| Probe 100 | chr16 | 1020768 | 1020888; |
| Probe 101 | chr16 | 1020873 | 1020993; |
| Probe 102 | chr16 | 1020978 | 1021098; |
| Probe 103 | chr16 | 1031125 | 1031245; |
| Probe 104 | chr16 | 1031230 | 1031350; |
| Probe 105 | chr16 | 1031335 | 1031455; |
| Probe 106 | chr16 | 1031440 | 1031560; |
| Probe 107 | chr16 | 1031545 | 1031665; |
| Probe 108 | chr16 | 1031650 | 1031770; |
| Probe 109 | chr16 | 1031755 | 1031875; |
| Probe 110 | chr16 | 1031860 | 1031980; |
| Probe 111 | chr16 | 1031965 | 1032085; |
| Probe 112 | chr16 | 1032070 | 1032190; |
| Probe 113 | chr19 | 45448788 | 45448908; |
| Probe 114 | chr19 | 45448893 | 45449013; |
| Probe 115 | chr19 | 45448998 | 45449118; |
| Probe 116 | chr19 | 45449103 | 45449223; |
| Probe 117 | chr19 | 45449208 | 45449328; |
| Probe 118 | chr19 | 45449313 | 45449433; |
| Probe 119 | chr19 | 45449418 | 45449538; |
| Probe 120 | chr19 | 45449628 | 45449748; |
| Probe 121 | chr19 | 45449733 | 45449853; |
| Probe 122 | chr19 | 45450048 | 45450168; |
| Probe 123 | chr19 | 45450363 | 45450483; |
| Probe 124 | chr19 | 45450468 | 45450588; |
| Probe 125 | chr19 | 45450573 | 45450693; |
| Probe 126 | chr19 | 45450678 | 45450798; |
| Probe 127 | chr19 | 45451518 | 45451638; |
| Probe 128 | chr19 | 45451623 | 45451743; |
| Probe 129 | chr19 | 45451728 | 45451848; |
| Probe 130 | chr19 | 45451833 | 45451953; |
| Probe 131 | chr19 | 45451938 | 45452058; |
| Probe 132 | chr19 | 45452043 | 45452163; |
| Probe 133 | chr19 | 45452148 | 45452268; |
| Probe 134 | chr19 | 45452253 | 45452373; |
| Probe 135 | chr19 | 45452358 | 45452478; |
| Probe 136 | chr19 | 45452463 | 45452583; |
| Probe 137 | chr19 | 45452568 | 45452688; |
| Probe 138 | chr19 | 45452673 | 45452793; |
| Probe 139 | chr19 | 45452778 | 45452898; |
| Probe 140 | chr8 | 19796244 | 19796364; |
| Probe 141 | chr8 | 19796349 | 19796469; |
| Probe 142 | chr8 | 19796454 | 19796574; |
| Probe 143 | chr8 | 19796559 | 19796679; |
| Probe 144 | chr8 | 19796664 | 19796784; |
| Probe 145 | chr8 | 19796769 | 19796889; |
| Probe 146 | chr8 | 19796874 | 19796994; |
| Probe 147 | chr8 | 19796979 | 19797099; |
| Probe 148 | chr8 | 19797084 | 19797204; |
| Probe 149 | chr8 | 19797189 | 19797309; |
| Probe 150 | chr8 | 19797294 | 19797414; |
| Probe 151 | chr8 | 19797399 | 19797519; |
| Probe 152 | chr8 | 19797504 | 19797624; |
| Probe 153 | chr8 | 19797609 | 19797729; |
| Probe 154 | chr8 | 19797714 | 19797834; |
| Probe 155 | chr8 | 19797819 | 19797939; |
| Probe 156 | chr8 | 19797924 | 19798044; |
| Probe 157 | chr8 | 19798029 | 19798149; |
| Probe 158 | chr8 | 19798134 | 19798254; |
| Probe 159 | chr8 | 19798239 | 19798359; |
| Probe 160 | chr8 | 19798344 | 19798464; |
| Probe 161 | chr8 | 19798449 | 19798569; |
| Probe 162 | chr8 | 19798554 | 19798674; |
| Probe 163 | chr8 | 19798659 | 19798779; |
| Probe 164 | chr8 | 19798764 | 19798884; |
| Probe 165 | chr8 | 19798869 | 19798989; |
| Probe 166 | chr8 | 19798974 | 19799094; |
| Probe 167 | chr8 | 19799079 | 19799199; |
| Probe 168 | chr8 | 19799184 | 19799304; |
| Probe 169 | chr8 | 19799289 | 19799409; |
| Probe 170 | chr8 | 19799394 | 19799514; |
| Probe 171 | chr8 | 19799499 | 19799619; |
| Probe 172 | chr8 | 19799604 | 19799724; |
| Probe 173 | chr8 | 19799709 | 19799829; |
| Probe 174 | chr8 | 19799814 | 19799934; |
| Probe 175 | chr8 | 19799919 | 19800039; |
| Probe 176 | chr8 | 19800024 | 19800144; |
| Probe 177 | chr8 | 19800129 | 19800249; |
| Probe 178 | chr8 | 19800234 | 19800354; |
| Probe 179 | chr8 | 19800339 | 19800459; |
| Probe 180 | chr8 | 19800444 | 19800564; |
| Probe 181 | chr8 | 19800549 | 19800669; |
| Probe 182 | chr8 | 19800654 | 19800774; |
| Probe 183 | chr8 | 19800759 | 19800879; |
| Probe 184 | chr8 | 19800864 | 19800984; |
| Probe 185 | chr8 | 19800969 | 19801089; |
| Probe 186 | chr8 | 19801074 | 19801194; |
| Probe 187 | chr8 | 19801179 | 19801299; |
| Probe 188 | chr8 | 19801284 | 19801404; |
| Probe 189 | chr8 | 19801704 | 19801824; |
| Probe 190 | chr8 | 19801809 | 19801929; |
| Probe 191 | chr8 | 19801914 | 19802034; |
| Probe 192 | chr8 | 19802019 | 19802139; |
| Probe 193 | chr8 | 19802124 | 19802244; |
| Probe 194 | chr8 | 19802229 | 19802349; |
| Probe 195 | chr8 | 19802334 | 19802454; |
| Probe 196 | chr8 | 19802439 | 19802559; |
| Probe 197 | chr8 | 19802544 | 19802664; |
| Probe 198 | chr8 | 19802649 | 19802769; |
| Probe 199 | chr8 | 19802754 | 19802874; |
| Probe 200 | chr8 | 19802859 | 19802979; |
| Probe 201 | chr8 | 19802964 | 19803084; |
| Probe 202 | chr8 | 19803069 | 19803189; |
| Probe 203 | chr8 | 19803174 | 19803294; |
| Probe 204 | chr8 | 19803279 | 19803399; |
| Probe 205 | chr8 | 19803384 | 19803504; |
| Probe 206 | chr8 | 19803489 | 19803609; |
| Probe 207 | chr8 | 19803594 | 19803714; |
| Probe 208 | chr8 | 19803699 | 19803819; |
| Probe 209 | chr8 | 19803804 | 19803924; |
| Probe 210 | chr8 | 19803909 | 19804029; |
| Probe 211 | chr8 | 19804014 | 19804134; |
| Probe 212 | chr8 | 19804119 | 19804239; |
| Probe 213 | chr8 | 19804224 | 19804344; |
| Probe 214 | chr8 | 19804329 | 19804449; |
| Probe 215 | chr8 | 19804434 | 19804554; |
| Probe 216 | chr8 | 19804539 | 19804659; |
| Probe 217 | chr8 | 19804644 | 19804764; |
| Probe 218 | chr8 | 19805064 | 19805184; |
| Probe 219 | chr8 | 19805169 | 19805289; |
| Probe 220 | chr8 | 19805274 | 19805394; |
| Probe 221 | chr8 | 19805379 | 19805499; |
| Probe 222 | chr8 | 19805484 | 19805604; |
| Probe 223 | chr8 | 19805589 | 19805709; |
| Probe 224 | chr8 | 19805694 | 19805814; |
| Probe 225 | chr8 | 19805799 | 19805919; |
| Probe 226 | chr8 | 19805904 | 19806024; |
| Probe 227 | chr8 | 19806009 | 19806129; |
| Probe 228 | chr8 | 19806114 | 19806234; |
| Probe 229 | chr8 | 19806219 | 19806339; |
| Probe 230 | chr8 | 19806324 | 19806444; |
| Probe 231 | chr8 | 19806429 | 19806549; |
| Probe 232 | chr8 | 19806534 | 19806654; |
| Probe 233 | chr8 | 19806639 | 19806759; |
| Probe 234 | chr8 | 19806744 | 19806864; |
| Probe 235 | chr8 | 19806849 | 19806969; |
| Probe 236 | chr8 | 19807269 | 19807389; |
| Probe 237 | chr8 | 19807374 | 19807494; |
| Probe 238 | chr8 | 19807479 | 19807599; |
| Probe 239 | chr8 | 19807584 | 19807704; |
| Probe 240 | chr8 | 19807689 | 19807809; |
| Probe 241 | chr8 | 19807794 | 19807914; |
| Probe 242 | chr8 | 19807899 | 19808019; |
| Probe 243 | chr8 | 19808319 | 19808439; |
| Probe 244 | chr8 | 19808424 | 19808544; |
| Probe 245 | chr8 | 19808529 | 19808649; |
| Probe 246 | chr8 | 19808634 | 19808754; |
| Probe 247 | chr8 | 19808739 | 19808859; |
| Probe 248 | chr8 | 19808844 | 19808964; |
| Probe 249 | chr8 | 19808949 | 19809069; |
| Probe 250 | chr8 | 19809054 | 19809174; |
| Probe 251 | chr8 | 19809159 | 19809279; |
| Probe 252 | chr8 | 19809264 | 19809384; |
| Probe 253 | chr8 | 19809369 | 19809489; |
| Probe 254 | chr8 | 19809474 | 19809594; |
| Probe 255 | chr8 | 19809579 | 19809699; |
| Probe 256 | chr8 | 19809684 | 19809804; |
| Probe 257 | chr8 | 19809789 | 19809909; |
| Probe 258 | chr8 | 19809894 | 19810014; |
| Probe 259 | chr8 | 19809999 | 19810119; |
| Probe 260 | chr8 | 19810104 | 19810224; |
| Probe 261 | chr8 | 19810209 | 19810329; |
| Probe 262 | chr8 | 19810314 | 19810434; |
| Probe 263 | chr8 | 19810419 | 19810539; |
| Probe 264 | chr8 | 19810524 | 19810644; |
| Probe 265 | chr8 | 19810629 | 19810749; |
| Probe 266 | chr8 | 19810734 | 19810854; |
| Probe 267 | chr8 | 19810839 | 19810959; |
| Probe 268 | chr8 | 19810944 | 19811064; |
| Probe 269 | chr8 | 19811049 | 19811169; |
| Probe 270 | chr8 | 19811154 | 19811274; |
| Probe 271 | chr8 | 19811259 | 19811379; |
| Probe 272 | chr8 | 19811364 | 19811484; |
| Probe 273 | chr8 | 19811469 | 19811589; |
| Probe 274 | chr8 | 19811574 | 19811694; |
| Probe 275 | chr8 | 19811679 | 19811799; |
| Probe 276 | chr8 | 19811784 | 19811904; |
| Probe 277 | chr8 | 19811889 | 19812009; |
| Probe 278 | chr8 | 19811994 | 19812114; |
| Probe 279 | chr8 | 19812099 | 19812219; |
| Probe 280 | chr8 | 19812204 | 19812324; |
| Probe 281 | chr8 | 19812309 | 19812429; |
| Probe 282 | chr8 | 19812519 | 19812639; |
| Probe 283 | chr8 | 19812624 | 19812744; |
| Probe 284 | chr8 | 19812729 | 19812849; |
| Probe 285 | chr8 | 19812834 | 19812954; |
| Probe 286 | chr8 | 19812939 | 19813059; |
| Probe 287 | chr8 | 19813044 | 19813164; |
| Probe 288 | chr8 | 19813149 | 19813269; |
| Probe 289 | chr8 | 19813254 | 19813374; |
| Probe 290 | chr8 | 19813359 | 19813479; |
| Probe 291 | chr8 | 19813464 | 19813584; |
| Probe 292 | chr8 | 19813569 | 19813689; |
| Probe 293 | chr8 | 19813674 | 19813794; |
| Probe 294 | chr8 | 19813779 | 19813899; |
| Probe 295 | chr8 | 19813884 | 19814004; |
| Probe 296 | chr8 | 19814094 | 19814214; |
| Probe 297 | chr8 | 19814199 | 19814319; |
| Probe 298 | chr8 | 19814304 | 19814424; |
| Probe 299 | chr8 | 19814619 | 19814739; |
| Probe 300 | chr8 | 19814724 | 19814844; |
| Probe 301 | chr8 | 19814829 | 19814949; |
| Probe 302 | chr8 | 19814934 | 19815054; |
| Probe 303 | chr8 | 19815039 | 19815159; |
| Probe 304 | chr8 | 19815144 | 19815264; |
| Probe 305 | chr8 | 19815249 | 19815369; |
| Probe 306 | chr8 | 19815354 | 19815474; |
| Probe 307 | chr8 | 19815459 | 19815579; |
| Probe 308 | chr8 | 19815774 | 19815894; |
| Probe 309 | chr8 | 19815879 | 19815999; |
| Probe 310 | chr8 | 19815984 | 19816104; |
| Probe 311 | chr8 | 19816194 | 19816314; |
| Probe 312 | chr8 | 19816299 | 19816419; |
| Probe 313 | chr8 | 19816404 | 19816524; |
| Probe 314 | chr8 | 19816509 | 19816629; |
| Probe 315 | chr8 | 19816614 | 19816734; |
| Probe 316 | chr8 | 19816719 | 19816839; |
| Probe 317 | chr8 | 19816824 | 19816944; |
| Probe 318 | chr8 | 19816929 | 19817049; |
| Probe 319 | chr8 | 19817034 | 19817154; |
| Probe 320 | chr8 | 19817139 | 19817259; |
| Probe 321 | chr8 | 19817559 | 19817679; |
| Probe 322 | chr8 | 19817664 | 19817784; |
| Probe 323 | chr8 | 19817769 | 19817889; |
| Probe 324 | chr8 | 19817874 | 19817994; |
| Probe 325 | chr8 | 19817979 | 19818099; |
| Probe 326 | chr8 | 19818084 | 19818204; |
| Probe 327 | chr8 | 19818189 | 19818309; |
| Probe 328 | chr8 | 19818294 | 19818414; |
| Probe 329 | chr8 | 19818399 | 19818519; |
| Probe 330 | chr8 | 19818504 | 19818624; |
| Probe 331 | chr8 | 19818609 | 19818729; |
| Probe 332 | chr8 | 19818714 | 19818834; |
| Probe 333 | chr8 | 19818819 | 19818939; |
| Probe 334 | chr8 | 19818924 | 19819044; |
| Probe 335 | chr8 | 19819029 | 19819149; |
| Probe 336 | chr8 | 19819134 | 19819254; |
| Probe 337 | chr8 | 19819239 | 19819359; |
| Probe 338 | chr8 | 19819344 | 19819464; |
| Probe 339 | chr8 | 19819449 | 19819569; |
| Probe 340 | chr8 | 19819554 | 19819674; |
| Probe 341 | chr8 | 19819659 | 19819779; |
| Probe 342 | chr8 | 19819764 | 19819884; |
| Probe 343 | chr8 | 19819869 | 19819989; |
| Probe 344 | chr8 | 19819974 | 19820094; |
| Probe 345 | chr8 | 19820079 | 19820199; |
| Probe 346 | chr8 | 19820184 | 19820304; |
| Probe 347 | chr8 | 19820289 | 19820409; |
| Probe 348 | chr8 | 19820394 | 19820514; |
| Probe 349 | chr8 | 19820499 | 19820619; |
| Probe 350 | chr8 | 19820604 | 19820724; |
| Probe 351 | chr8 | 19820919 | 19821039; |
| Probe 352 | chr8 | 19821024 | 19821144; |
| Probe 353 | chr8 | 19821129 | 19821249; |
| Probe 354 | chr8 | 19821234 | 19821354; |
| Probe 355 | chr8 | 19821339 | 19821459; |
| Probe 356 | chr8 | 19821759 | 19821879; |
| Probe 357 | chr8 | 19821864 | 19821984; |
| Probe 358 | chr8 | 19821969 | 19822089; |
| Probe 359 | chr8 | 19822284 | 19822404; |
| Probe 360 | chr8 | 19822389 | 19822509; |
| Probe 361 | chr8 | 19822494 | 19822614; |
| Probe 362 | chr8 | 19822599 | 19822719; |
| Probe 363 | chr8 | 19822704 | 19822824; |
| Probe 364 | chr8 | 19822809 | 19822929; |
| Probe 365 | chr8 | 19822914 | 19823034; |
| Probe 366 | chr8 | 19823019 | 19823139; |
| Probe 367 | chr8 | 19823124 | 19823244; |
| Probe 368 | chr8 | 19823229 | 19823349; |
| Probe 369 | chr8 | 19823334 | 19823454; |
| Probe 370 | chr8 | 19823439 | 19823559; |
| Probe 371 | chr8 | 19823544 | 19823664; |
| Probe 372 | chr8 | 19823649 | 19823769; |
| Probe 373 | chr8 | 19823754 | 19823874; |
| Probe 374 | chr8 | 19823859 | 19823979; |
| Probe 375 | chr8 | 19823964 | 19824084; |
| Probe 376 | chr8 | 19824069 | 19824189; |
| Probe 377 | chr8 | 19824174 | 19824294; |
| Probe 378 | chr8 | 19824279 | 19824399; |
| Probe 379 | chr8 | 19824384 | 19824504; |
| Probe 380 | chr8 | 19824489 | 19824609; |
| Probe 381 | chr8 | 19824594 | 19824714; |
| Probe 382 | chr8 | 19824699 | 19824819; |
| Probe 383 | chr8 | 144294573 | 144294693; |
| Probe 384 | chr8 | 144294678 | 144294798; |
| Probe 385 | chr8 | 144294783 | 144294903; |
| Probe 386 | chr8 | 144294888 | 144295008; |
| Probe 387 | chr8 | 144294993 | 144295113; |
| Probe 388 | chr8 | 144295098 | 144295218; |
| Probe 389 | chr8 | 144295203 | 144295323; |
| Probe 390 | chr8 | 144295308 | 144295428; |
| Probe 391 | chr8 | 144295413 | 144295533; |
| Probe 392 | chr8 | 144295518 | 144295638; |
| Probe 393 | chr8 | 144295623 | 144295743; |
| Probe 394 | chr8 | 144295728 | 144295848; |
| Probe 395 | chr8 | 144295833 | 144295953; |
| Probe 396 | chr8 | 144295938 | 144296058; |
| Probe 397 | chr8 | 144296043 | 144296163; |
| Probe 398 | chr8 | 144296148 | 144296268; |
| Probe 399 | chr8 | 144296253 | 144296373; |
| Probe 400 | chr8 | 144296358 | 144296478; |
| Probe 401 | chr8 | 144296463 | 144296583; |
| Probe 402 | chr8 | 144296568 | 144296688; |
| Probe 403 | chr8 | 144296673 | 144296793; |
| Probe 404 | chr8 | 144296778 | 144296898; |
| Probe 405 | chr8 | 144296883 | 144297003; |
| Probe 406 | chr8 | 144296988 | 144297108; |
| Probe 407 | chr8 | 144297093 | 144297213; |
| Probe 408 | chr8 | 144297198 | 144297318; |
| Probe 409 | chr8 | 144297303 | 144297423; |
| Probe 410 | chr8 | 144297408 | 144297528; |
| Probe 411 | chr8 | 144297513 | 144297633; |
| Probe 412 | chr8 | 144297618 | 144297738; |
| Probe 413 | chr8 | 144297723 | 144297843; |
| Probe 414 | chr8 | 144297828 | 144297948; |
| Probe 415 | chr8 | 144297933 | 144298053; |
| Probe 416 | chr8 | 144298038 | 144298158; |
| Probe 417 | chr8 | 144298143 | 144298263; |
| Probe 418 | chr8 | 144298248 | 144298368; |
| Probe 419 | chr8 | 144298353 | 144298473; |
| Probe 420 | chr8 | 144298458 | 144298578; |
| Probe 421 | chr8 | 144298563 | 144298683; |
| Probe 422 | chr8 | 144298668 | 144298788; |
| Probe 423 | chr8 | 144298773 | 144298893; |
| Probe 424 | chr8 | 144298878 | 144298998; |
| Probe 425 | chr8 | 144298983 | 144299103; |

The reference genome is Hg19.

In some specific embodiments of the present application, the capture probes comprise a plurality of probe sets selected from a probe set 1 to a probe set 6;
The probe set 1 comprises a plurality of probes selected from the probes 1 to 26;
The probe set 2 comprises a plurality of probes selected from the probes 27 to 64;
The probe set 3 comprises a plurality of probes selected from the probes 65 to 112;
The probe set 4 comprises a plurality of probes selected from the probes 113 to 139;
The probe set 5 comprises a plurality of probes selected from the probes 140 to 382;
The probe set 6 comprises a plurality of probes selected from the probes 383 to 425.

In some specific embodiments of the present application, the capture probes comprise the probe set 1 to the probe set 6;
The probe set 1 comprises the probes 1 to 26;
The probe set 2 comprises the probes 27 to 64;
The probe set 3 comprises the probes 65 to 112;
The probe set 4 comprises the probes 113 to 139;
The probe set 5 comprises the probes 140 to 382;
The probe set 6 comprises the probes 383 to 425.

In another embodiment of the present application, the capture probes are used for preparing a familial chylomicronemia syndrome detection kit.

In still another embodiment of the present application, a familial chylomicronemia syndrome detection kit comprising the capture probes is provided.

In some specific embodiments of the present application, the familial chylomicronemia syndrome detection kit further includes one or more of a nucleic acid extraction reagent, a DNA library construction reagent, a sample and library quantification reagent, a fragment quality control reagent, a hybridization capture reagent, a nucleic acid purification reagent, a target gene fragment amplification reagent, and a sequencing reagent.

In yet another one or more embodiments of the present application, use of the capture probes for constructing a detection library for familial chylomicronemia syndrome-related gene mutations is provided.

In a further one or more embodiments of the present application, use of the detection kit for constructing a detection library for familial chylomicronemia syndrome-related gene mutations is provided.

In a further one or more embodiments of the present application, a method for detecting familial chylomicronemia syndrome-related gene mutations is provided, comprising capturing target gene fragments containing the mutations by using the capture probes or the detection kit, and detecting the captured fragments.

In some specific embodiments of the present application, sequencing is used to detect the captured fragments.

Details of one or more embodiments of the present application are presented in the following description, and other features, objectives, and advantages of the present application will become apparent from the description and the claims.

### Specific Embodiments

Below, the present invention is further described in detail in conjunction with embodiments and examples. It should be understood that these embodiments and examples are only used to illustrate the present invention and are not used to limit the scope of the present invention, and the purpose of providing these embodiments and examples is to make the understanding of the disclosed content of the present invention more thorough and comprehensive. It should also be understood that the present invention can be realized in many different forms and is not limited to the embodiments and examples described herein, and those skilled in the art can make various changes or modifications without departing from the substance of the present invention, and any equivalent forms obtained in a similar way shall fall within the protection scope of the present application. In addition, in the following description, a large number of specific details are given in order to facilitate a more adequate understanding of the present invention, and it should be understood that the present invention may be implemented without one or more of these details.

Unless otherwise defined, all technical and scientific terms used herein have the same meanings as are commonly understood by those skilled in the technical field to which the present invention belongs. The terms used herein in the specification of the present invention are only for the purpose of describing the embodiments and examples, and are not intended to limit the present invention.

### Terms

Unless otherwise stated or where there is a contradiction, the terms or phrases used herein have the following meanings:
The scope of selection of the terms "and/or," "or/and," and "as well as/or" used herein includes any one item among two or more related listed items, and also includes any and all combinations of the related listed items, wherein the any and all combinations include a combination of any two related listed items, any more than two related listed items, or all related listed items. It should be noted that when at least three items are connected by a combination of at least two conjunctions selected from "and/or," "or/and," and "as well as/or," it should be understood that, in the present application, the technical solution undoubtedly includes the technical solution in which all are connected by "logical AND," and also undoubtedly includes the technical solution in which all are connected by "logical OR." For example, "A and/or B" includes three alternatives: A, B, and A+B. For another example, the technical solution of "A, as well as/or, B, as well as/or, C, as well as/or, D" includes any one of A, B, C, and D (that is, the technical solution in which all are connected by "logical OR"), and also includes any and all combinations of A, B, C, and D, that is, includes a combination of any two items or any three items among A, B, C, and D, and further includes the four-item combination of A, B, C, and D (that is, the technical solution in which all are connected by "logical AND").

In the present invention, terms such as "a plurality of," "multiple kinds," "multiple times," and "multiple elements," unless otherwise specifically limited, mean greater than 2 or equal to 2 in quantity. For example, "one or more kinds" means one kind or two or more kinds.

"Combination thereof," "any combination thereof," "any combination manner thereof," and the like used herein include all suitable combination manners of any two items or any more than two items among the listed items.

Herein, the term "suitable" as in "suitable combination manner," "suitable manner," "any suitable manner," and the like is based on whether the technical solution of the present invention can be implemented, the technical problem of the present invention can be solved, and the intended technical effect of the present invention can be achieved.

Herein, "preferably," "better," "more preferably," and "as appropriate" are only used to describe embodiments or examples with better effects, and it should be understood that they do not constitute a limitation on the protection scope of the present invention.

In the present invention, "further," "still further," "particularly," and the like are used for descriptive purposes and indicate differences in content, but should not be understood as limitations on the protection scope of the present invention.

In the present invention, "optionally," "optional," and "option" mean may or may not be present, that is, mean any one selected from the two parallel solutions of "present" or "absent." If a technical solution contains multiple occurrences of "optional," then, unless otherwise specifically stated and where there is no contradiction or mutually restrictive relationship, each "optional" is independent of the others.

In the present invention, in "a first aspect," "a second aspect," "a third aspect," "a fourth aspect," and the like, the terms "first," "second," "third," "fourth," and the like are only used for descriptive purposes, and cannot be understood as indicating or implying relative importance or quantity, nor can they be understood as implicitly indicating the importance or quantity of the indicated technical features. Moreover, "first," "second," "third," "fourth," and the like only serve the purpose of non-exhaustive enumerative description, and should be understood as not constituting a closed-ended limitation on quantity.

In the present invention, among technical features described in an open-ended manner, included are closed-ended technical solutions composed of the recited features, and also included are open-ended technical solutions comprising the recited features.

In the present invention, where a numerical interval (that is, a numerical range) is involved, unless otherwise specifically stated, the optional numerical values distributed within the above numerical interval are regarded as continuous, and include the two numerical endpoints of the numerical range (that is, the minimum value and the maximum value), as well as each numerical value between the two numerical endpoints. Unless otherwise specifically stated, when a numerical interval refers only to integers within the numerical interval, including the two endpoint integers of the numerical range, as well as each integer between the two endpoints, this is equivalent herein to directly listing each integer, for example, t being an integer selected from 1 to 10 means that t is any one integer selected from the integer group consisting of 1, 2, 3, 4, 5, 6, 7, 8, 9, and 10. In addition, when multiple ranges describe features or characteristics, these ranges may be combined. In other words, unless otherwise indicated, the ranges disclosed herein should be understood as including any and all subranges subsumed therein.

The temperature parameters in the present invention, unless otherwise specifically limited, are allowed to be constant-temperature treatment, and are also allowed to vary within a certain temperature interval. It should be understood that the constant-temperature treatment allows the temperature to fluctuate within the precision range controlled by the instrument. Fluctuation is allowed within a range such as ±5°C, ±4°C, ±3°C, ±2°C, or ±1°C.

In the present invention, % (w/w) and wt% both denote weight percentage, % (v/v) denotes volume percentage, and % (w/v) denotes mass/volume percentage.

All literature mentioned in the present invention is incorporated herein by reference, just as if each of the literature were individually incorporated by reference. Unless there is a conflict with the inventive purpose and/or technical solution of the present application, the cited literature involved in the present invention is cited for its entire content and all purposes. When cited literature is involved in the present invention, the definitions of related technical features, terms, nouns, phrases, and the like in the cited literature are also incorporated. When cited literature is involved in the present invention, examples and preferred modes of the cited related technical features may also be incorporated into the present application as reference, but only to the extent that the present invention can be implemented. It should be understood that, when the cited content conflicts with the description in the present application, the present application shall prevail or adaptive correction may be made according to the description of the present application.

The embodiments of the present invention will be described in detail below in conjunction with examples. It should be understood that these examples are only used to illustrate the present invention and are not used to limit the scope of the present invention. For the experimental methods in the following examples for which specific conditions are not indicated, the guidance provided in the present invention shall be consulted first, and they may also be based on experimental manuals or conventional conditions in the art, may also follow the recommendation of the manufacturer, or may be based on experimental methods known in the art.

FCS is caused by one or more gene mutations, and these gene mutations can cause loss of gene functions in lipoprotein lipase (LPL), apolipoprotein CII (APOC2), lipase maturation factor 1 (LMF1), glycosylphosphatidylinositol-anchored high density lipoprotein-binding protein 1 (GPIHBP1), apolipoprotein AV (APOA5), and glycerol-3-phosphate dehydrogenase 1 (GPD1), thereby affecting the lipolysis and clearance of chylomicrons. In one example embodiment of the present application, 6 genes closely related to FCS were selected (*LPL*, *APOC2*, *LMF1*, *GPIHBP1*, *APOA5*, and *GPD1*), and a probe panel covering exon and hotspot intron regions of the 6 genes was designed for capture sequencing, aiming to stably and accurately detect gene mutations related to FCS, and to provide a more convenient and accurate FCS detection method for clinical practice. It has important significance for the clinical diagnosis and treatment of FCS.

### In a first aspect of the embodiments of the present application

The present application provides a capture probe comprising one or more probe sets selected from a probe set 1 to a probe set 6;
Probe set 1 comprises one or more probes selected from probes 1 to 26;
Probe set 2 comprises one or more probes selected from probes 27 to 64;
Probe set 3 comprises one or more probes selected from probes 65 to 112;
Probe set 4 comprises one or more probes selected from probes 113 to 139;
Probe set 5 comprises one or more probes selected from probes 140 to 382;
Probe set 6 comprises one or more probes selected from probes 383 to 425;
The probes 1 to 425 are constructed for target gene fragments located between different start positions and end positions on the following chromosomes and are complementary to and paired with said target gene fragments:

| Probe number | Chromosome | Start position | End position |
|---|---|---|---|
| Probe 1 | chr11 | 116660063 | 116660183; |
| Probe 2 | chr11 | 116660168 | 116660288; |
| Probe 3 | chr11 | 116660273 | 116660393; |
| Probe 4 | chr11 | 116660378 | 116660498; |
| Probe 5 | chr11 | 116660483 | 116660603; |
| Probe 6 | chr11 | 116660588 | 116660708; |
| Probe 7 | chr11 | 116660693 | 116660813; |
| Probe 8 | chr11 | 116660798 | 116660918; |
| Probe 9 | chr11 | 116660903 | 116661023; |
| Probe 10 | chr11 | 116661008 | 116661128; |
| Probe 11 | chr11 | 116661113 | 116661233; |
| Probe 12 | chr11 | 116661218 | 116661338; |
| Probe 13 | chr11 | 116661323 | 116661443; |
| Probe 14 | chr11 | 116661428 | 116661548; |
| Probe 15 | chr11 | 116661533 | 116661653; |
| Probe 16 | chr11 | 116661638 | 116661758; |
| Probe 17 | chr11 | 116661743 | 116661863; |
| Probe 18 | chr11 | 116662282 | 116662402; |
| Probe 19 | chr11 | 116662387 | 116662507; |
| Probe 20 | chr11 | 116662508 | 116662628; |
| Probe 21 | chr11 | 116663076 | 116663196; |
| Probe 22 | chr11 | 116663181 | 116663301; |
| Probe 23 | chr11 | 116663286 | 116663406; |
| Probe 24 | chr11 | 116663391 | 116663511; |
| Probe 25 | chr11 | 116663496 | 116663616; |
| Probe 26 | chr11 | 116663601 | 116663721; |
| Probe 27 | chr12 | 50497271 | 50497391; |
| Probe 28 | chr12 | 50497376 | 50497496; |
| Probe 29 | chr12 | 50497481 | 50497601; |
| Probe 30 | chr12 | 50497586 | 50497706; |
| Probe 31 | chr12 | 50497691 | 50497811; |
| Probe 32 | chr12 | 50497796 | 50497916; |
| Probe 33 | chr12 | 50498337 | 50498457; |
| Probe 34 | chr12 | 50498442 | 50498562; |
| Probe 35 | chr12 | 50499311 | 50499431; |
| Probe 36 | chr12 | 50499416 | 50499536; |
| Probe 37 | chr12 | 50500051 | 50500171; |
| Probe 38 | chr12 | 50500156 | 50500276; |
| Probe 39 | chr12 | 50500568 | 50500688; |
| Probe 40 | chr12 | 50500673 | 50500793; |
| Probe 41 | chr12 | 50501330 | 50501450; |
| Probe 42 | chr12 | 50501435 | 50501555; |
| Probe 43 | chr12 | 50501540 | 50501660; |
| Probe 44 | chr12 | 50501787 | 50501907; |
| Probe 45 | chr12 | 50501892 | 50502012; |
| Probe 46 | chr12 | 50503186 | 50503306; |
| Probe 47 | chr12 | 50503291 | 50503411; |
| Probe 48 | chr12 | 50503396 | 50503516; |
| Probe 49 | chr12 | 50503501 | 50503621; |
| Probe 50 | chr12 | 50503606 | 50503726; |
| Probe 51 | chr12 | 50503711 | 50503831; |
| Probe 52 | chr12 | 50503816 | 50503936; |
| Probe 53 | chr12 | 50503921 | 50504041; |
| Probe 54 | chr12 | 50504026 | 50504146; |
| Probe 55 | chr12 | 50504131 | 50504251; |
| Probe 56 | chr12 | 50504236 | 50504356; |
| Probe 57 | chr12 | 50504341 | 50504461; |
| Probe 58 | chr12 | 50504446 | 50504566; |
| Probe 59 | chr12 | 50504551 | 50504671; |
| Probe 60 | chr12 | 50504656 | 50504776; |
| Probe 61 | chr12 | 50504761 | 50504881; |
| Probe 62 | chr12 | 50504866 | 50504986; |
| Probe 63 | chr12 | 50504971 | 50505091; |
| Probe 64 | chr12 | 50505076 | 50505196; |
| Probe 65 | chr16 | 903614 | 903734; |
| Probe 66 | chr16 | 903719 | 903839; |
| Probe 67 | chr16 | 903824 | 903944; |
| Probe 68 | chr16 | 903929 | 904049; |
| Probe 69 | chr16 | 904034 | 904154; |
| Probe 70 | chr16 | 904139 | 904259; |
| Probe 71 | chr16 | 904244 | 904364; |
| Probe 72 | chr16 | 904349 | 904469; |
| Probe 73 | chr16 | 904454 | 904574; |
| Probe 74 | chr16 | 904559 | 904679; |
| Probe 75 | chr16 | 904664 | 904784; |
| Probe 76 | chr16 | 905087 | 905207; |
| Probe 77 | chr16 | 905192 | 905312; |
| Probe 78 | chr16 | 918924 | 919044; |
| Probe 79 | chr16 | 919029 | 919149; |
| Probe 80 | chr16 | 919863 | 919983; |
| Probe 81 | chr16 | 919968 | 920088; |
| Probe 82 | chr16 | 920709 | 920829; |
| Probe 83 | chr16 | 920814 | 920934; |
| Probe 84 | chr16 | 921141 | 921261; |
| Probe 85 | chr16 | 921246 | 921366; |
| Probe 86 | chr16 | 929550 | 929670; |
| Probe 87 | chr16 | 929655 | 929775; |
| Probe 88 | chr16 | 942987 | 943107; |
| Probe 89 | chr16 | 960911 | 961031; |
| Probe 90 | chr16 | 961016 | 961136; |
| Probe 91 | chr16 | 981622 | 981742; |
| Probe 92 | chr16 | 981727 | 981847; |
| Probe 93 | chr16 | 983971 | 984091; |
| Probe 94 | chr16 | 984076 | 984196; |
| Probe 95 | chr16 | 984170 | 984290; |
| Probe 96 | chr16 | 1004337 | 1004457; |
| Probe 97 | chr16 | 1004442 | 1004562; |
| Probe 98 | chr16 | 1004547 | 1004667; |
| Probe 99 | chr16 | 1004652 | 1004772; |
| Probe 100 | chr16 | 1020768 | 1020888; |
| Probe 101 | chr16 | 1020873 | 1020993; |
| Probe 102 | chr16 | 1020978 | 1021098; |
| Probe 103 | chr16 | 1031125 | 1031245; |
| Probe 104 | chr16 | 1031230 | 1031350; |
| Probe 105 | chr16 | 1031335 | 1031455; |
| Probe 106 | chr16 | 1031440 | 1031560; |
| Probe 107 | chr16 | 1031545 | 1031665; |
| Probe 108 | chr16 | 1031650 | 1031770; |
| Probe 109 | chr16 | 1031755 | 1031875; |
| Probe 110 | chr16 | 1031860 | 1031980; |
| Probe 111 | chr16 | 1031965 | 1032085; |
| Probe 112 | chr16 | 1032070 | 1032190; |
| Probe 113 | chr19 | 45448788 | 45448908; |
| Probe 114 | chr19 | 45448893 | 45449013; |
| Probe 115 | chr19 | 45448998 | 45449118; |
| Probe 116 | chr19 | 45449103 | 45449223; |
| Probe 117 | chr19 | 45449208 | 45449328; |
| Probe 118 | chr19 | 45449313 | 45449433; |
| Probe 119 | chr19 | 45449418 | 45449538; |
| Probe 120 | chr19 | 45449628 | 45449748; |
| Probe 121 | chr19 | 45449733 | 45449853; |
| Probe 122 | chr19 | 45450048 | 45450168; |
| Probe 123 | chr19 | 45450363 | 45450483; |
| Probe 124 | chr19 | 45450468 | 45450588; |
| Probe 125 | chr19 | 45450573 | 45450693; |
| Probe 126 | chr19 | 45450678 | 45450798; |
| Probe 127 | chr19 | 45451518 | 45451638; |
| Probe 128 | chr19 | 45451623 | 45451743; |
| Probe 129 | chr19 | 45451728 | 45451848; |
| Probe 130 | chr19 | 45451833 | 45451953; |
| Probe 131 | chr19 | 45451938 | 45452058; |
| Probe 132 | chr19 | 45452043 | 45452163; |
| Probe 133 | chr19 | 45452148 | 45452268; |
| Probe 134 | chr19 | 45452253 | 45452373; |
| Probe 135 | chr19 | 45452358 | 45452478; |
| Probe 136 | chr19 | 45452463 | 45452583; |
| Probe 137 | chr19 | 45452568 | 45452688; |
| Probe 138 | chr19 | 45452673 | 45452793; |
| Probe 139 | chr19 | 45452778 | 45452898; |
| Probe 140 | chr8 | 19796244 | 19796364; |
| Probe 141 | chr8 | 19796349 | 19796469; |
| Probe 142 | chr8 | 19796454 | 19796574; |
| Probe 143 | chr8 | 19796559 | 19796679; |
| Probe 144 | chr8 | 19796664 | 19796784; |
| Probe 145 | chr8 | 19796769 | 19796889; |
| Probe 146 | chr8 | 19796874 | 19796994; |
| Probe 147 | chr8 | 19796979 | 19797099; |
| Probe 148 | chr8 | 19797084 | 19797204; |
| Probe 149 | chr8 | 19797189 | 19797309; |
| Probe 150 | chr8 | 19797294 | 19797414; |
| Probe 151 | chr8 | 19797399 | 19797519; |
| Probe 152 | chr8 | 19797504 | 19797624; |
| Probe 153 | chr8 | 19797609 | 19797729; |
| Probe 154 | chr8 | 19797714 | 19797834; |
| Probe 155 | chr8 | 19797819 | 19797939; |
| Probe 156 | chr8 | 19797924 | 19798044; |
| Probe 157 | chr8 | 19798029 | 19798149; |
| Probe 158 | chr8 | 19798134 | 19798254; |
| Probe 159 | chr8 | 19798239 | 19798359; |
| Probe 160 | chr8 | 19798344 | 19798464; |
| Probe 161 | chr8 | 19798449 | 19798569; |
| Probe 162 | chr8 | 19798554 | 19798674; |
| Probe 163 | chr8 | 19798659 | 19798779; |
| Probe 164 | chr8 | 19798764 | 19798884; |
| Probe 165 | chr8 | 19798869 | 19798989; |
| Probe 166 | chr8 | 19798974 | 19799094; |
| Probe 167 | chr8 | 19799079 | 19799199; |
| Probe 168 | chr8 | 19799184 | 19799304; |
| Probe 169 | chr8 | 19799289 | 19799409; |
| Probe 170 | chr8 | 19799394 | 19799514; |
| Probe 171 | chr8 | 19799499 | 19799619; |
| Probe 172 | chr8 | 19799604 | 19799724; |
| Probe 173 | chr8 | 19799709 | 19799829; |
| Probe 174 | chr8 | 19799814 | 19799934; |
| Probe 175 | chr8 | 19799919 | 19800039; |
| Probe 176 | chr8 | 19800024 | 19800144; |
| Probe 177 | chr8 | 19800129 | 19800249; |
| Probe 178 | chr8 | 19800234 | 19800354; |
| Probe 179 | chr8 | 19800339 | 19800459; |
| Probe 180 | chr8 | 19800444 | 19800564; |
| Probe 181 | chr8 | 19800549 | 19800669; |
| Probe 182 | chr8 | 19800654 | 19800774; |
| Probe 183 | chr8 | 19800759 | 19800879; |
| Probe 184 | chr8 | 19800864 | 19800984; |
| Probe 185 | chr8 | 19800969 | 19801089; |
| Probe 186 | chr8 | 19801074 | 19801194; |
| Probe 187 | chr8 | 19801179 | 19801299; |
| Probe 188 | chr8 | 19801284 | 19801404; |
| Probe 189 | chr8 | 19801704 | 19801824; |
| Probe 190 | chr8 | 19801809 | 19801929; |
| Probe 191 | chr8 | 19801914 | 19802034; |
| Probe 192 | chr8 | 19802019 | 19802139; |
| Probe 193 | chr8 | 19802124 | 19802244; |
| Probe 194 | chr8 | 19802229 | 19802349; |
| Probe 195 | chr8 | 19802334 | 19802454; |
| Probe 196 | chr8 | 19802439 | 19802559; |
| Probe 197 | chr8 | 19802544 | 19802664; |
| Probe 198 | chr8 | 19802649 | 19802769; |
| Probe 199 | chr8 | 19802754 | 19802874; |
| Probe 200 | chr8 | 19802859 | 19802979; |
| Probe 201 | chr8 | 19802964 | 19803084; |
| Probe 202 | chr8 | 19803069 | 19803189; |
| Probe 203 | chr8 | 19803174 | 19803294; |
| Probe 204 | chr8 | 19803279 | 19803399; |
| Probe 205 | chr8 | 19803384 | 19803504; |
| Probe 206 | chr8 | 19803489 | 19803609; |
| Probe 207 | chr8 | 19803594 | 19803714; |
| Probe 208 | chr8 | 19803699 | 19803819; |
| Probe 209 | chr8 | 19803804 | 19803924; |
| Probe 210 | chr8 | 19803909 | 19804029; |
| Probe 211 | chr8 | 19804014 | 19804134; |
| Probe 212 | chr8 | 19804119 | 19804239; |
| Probe 213 | chr8 | 19804224 | 19804344; |
| Probe 214 | chr8 | 19804329 | 19804449; |
| Probe 215 | chr8 | 19804434 | 19804554; |
| Probe 216 | chr8 | 19804539 | 19804659; |
| Probe 217 | chr8 | 19804644 | 19804764; |
| Probe 218 | chr8 | 19805064 | 19805184; |
| Probe 219 | chr8 | 19805169 | 19805289; |
| Probe 220 | chr8 | 19805274 | 19805394; |
| Probe 221 | chr8 | 19805379 | 19805499; |
| Probe 222 | chr8 | 19805484 | 19805604; |
| Probe 223 | chr8 | 19805589 | 19805709; |
| Probe 224 | chr8 | 19805694 | 19805814; |
| Probe 225 | chr8 | 19805799 | 19805919; |
| Probe 226 | chr8 | 19805904 | 19806024; |
| Probe 227 | chr8 | 19806009 | 19806129; |
| Probe 228 | chr8 | 19806114 | 19806234; |
| Probe 229 | chr8 | 19806219 | 19806339; |
| Probe 230 | chr8 | 19806324 | 19806444; |
| Probe 231 | chr8 | 19806429 | 19806549; |
| Probe 232 | chr8 | 19806534 | 19806654; |
| Probe 233 | chr8 | 19806639 | 19806759; |
| Probe 234 | chr8 | 19806744 | 19806864; |
| Probe 235 | chr8 | 19806849 | 19806969; |
| Probe 236 | chr8 | 19807269 | 19807389; |
| Probe 237 | chr8 | 19807374 | 19807494; |
| Probe 238 | chr8 | 19807479 | 19807599; |
| Probe 239 | chr8 | 19807584 | 19807704; |
| Probe 240 | chr8 | 19807689 | 19807809; |
| Probe 241 | chr8 | 19807794 | 19807914; |
| Probe 242 | chr8 | 19807899 | 19808019; |
| Probe 243 | chr8 | 19808319 | 19808439; |
| Probe 244 | chr8 | 19808424 | 19808544; |
| Probe 245 | chr8 | 19808529 | 19808649; |
| Probe 246 | chr8 | 19808634 | 19808754; |
| Probe 247 | chr8 | 19808739 | 19808859; |
| Probe 248 | chr8 | 19808844 | 19808964; |
| Probe 249 | chr8 | 19808949 | 19809069; |
| Probe 250 | chr8 | 19809054 | 19809174; |
| Probe 251 | chr8 | 19809159 | 19809279; |
| Probe 252 | chr8 | 19809264 | 19809384; |
| Probe 253 | chr8 | 19809369 | 19809489; |
| Probe 254 | chr8 | 19809474 | 19809594; |
| Probe 255 | chr8 | 19809579 | 19809699; |
| Probe 256 | chr8 | 19809684 | 19809804; |
| Probe 257 | chr8 | 19809789 | 19809909; |
| Probe 258 | chr8 | 19809894 | 19810014; |
| Probe 259 | chr8 | 19809999 | 19810119; |
| Probe 260 | chr8 | 19810104 | 19810224; |
| Probe 261 | chr8 | 19810209 | 19810329; |
| Probe 262 | chr8 | 19810314 | 19810434; |
| Probe 263 | chr8 | 19810419 | 19810539; |
| Probe 264 | chr8 | 19810524 | 19810644; |
| Probe 265 | chr8 | 19810629 | 19810749; |
| Probe 266 | chr8 | 19810734 | 19810854; |
| Probe 267 | chr8 | 19810839 | 19810959; |
| Probe 268 | chr8 | 19810944 | 19811064; |
| Probe 269 | chr8 | 19811049 | 19811169; |
| Probe 270 | chr8 | 19811154 | 19811274; |
| Probe 271 | chr8 | 19811259 | 19811379; |
| Probe 272 | chr8 | 19811364 | 19811484; |
| Probe 273 | chr8 | 19811469 | 19811589; |
| Probe 274 | chr8 | 19811574 | 19811694; |
| Probe 275 | chr8 | 19811679 | 19811799; |
| Probe 276 | chr8 | 19811784 | 19811904; |
| Probe 277 | chr8 | 19811889 | 19812009; |
| Probe 278 | chr8 | 19811994 | 19812114; |
| Probe 279 | chr8 | 19812099 | 19812219; |
| Probe 280 | chr8 | 19812204 | 19812324; |
| Probe 281 | chr8 | 19812309 | 19812429; |
| Probe 282 | chr8 | 19812519 | 19812639; |
| Probe 283 | chr8 | 19812624 | 19812744; |
| Probe 284 | chr8 | 19812729 | 19812849; |
| Probe 285 | chr8 | 19812834 | 19812954; |
| Probe 286 | chr8 | 19812939 | 19813059; |
| Probe 287 | chr8 | 19813044 | 19813164; |
| Probe 288 | chr8 | 19813149 | 19813269; |
| Probe 289 | chr8 | 19813254 | 19813374; |
| Probe 290 | chr8 | 19813359 | 19813479; |
| Probe 291 | chr8 | 19813464 | 19813584; |
| Probe 292 | chr8 | 19813569 | 19813689; |
| Probe 293 | chr8 | 19813674 | 19813794; |
| Probe 294 | chr8 | 19813779 | 19813899; |
| Probe 295 | chr8 | 19813884 | 19814004; |
| Probe 296 | chr8 | 19814094 | 19814214; |
| Probe 297 | chr8 | 19814199 | 19814319; |
| Probe 298 | chr8 | 19814304 | 19814424; |
| Probe 299 | chr8 | 19814619 | 19814739; |
| Probe 300 | chr8 | 19814724 | 19814844; |
| Probe 301 | chr8 | 19814829 | 19814949; |
| Probe 302 | chr8 | 19814934 | 19815054; |
| Probe 303 | chr8 | 19815039 | 19815159; |
| Probe 304 | chr8 | 19815144 | 19815264; |
| Probe 305 | chr8 | 19815249 | 19815369; |
| Probe 306 | chr8 | 19815354 | 19815474; |
| Probe 307 | chr8 | 19815459 | 19815579; |
| Probe 308 | chr8 | 19815774 | 19815894; |
| Probe 309 | chr8 | 19815879 | 19815999; |
| Probe 310 | chr8 | 19815984 | 19816104; |
| Probe 311 | chr8 | 19816194 | 19816314; |
| Probe 312 | chr8 | 19816299 | 19816419; |
| Probe 313 | chr8 | 19816404 | 19816524; |
| Probe 314 | chr8 | 19816509 | 19816629; |
| Probe 315 | chr8 | 19816614 | 19816734; |
| Probe 316 | chr8 | 19816719 | 19816839; |
| Probe 317 | chr8 | 19816824 | 19816944; |
| Probe 318 | chr8 | 19816929 | 19817049; |
| Probe 319 | chr8 | 19817034 | 19817154; |
| Probe 320 | chr8 | 19817139 | 19817259; |
| Probe 321 | chr8 | 19817559 | 19817679; |
| Probe 322 | chr8 | 19817664 | 19817784; |
| Probe 323 | chr8 | 19817769 | 19817889; |
| Probe 324 | chr8 | 19817874 | 19817994; |
| Probe 325 | chr8 | 19817979 | 19818099; |
| Probe 326 | chr8 | 19818084 | 19818204; |
| Probe 327 | chr8 | 19818189 | 19818309; |
| Probe 328 | chr8 | 19818294 | 19818414; |
| Probe 329 | chr8 | 19818399 | 19818519; |
| Probe 330 | chr8 | 19818504 | 19818624; |
| Probe 331 | chr8 | 19818609 | 19818729; |
| Probe 332 | chr8 | 19818714 | 19818834; |
| Probe 333 | chr8 | 19818819 | 19818939; |
| Probe 334 | chr8 | 19818924 | 19819044; |
| Probe 335 | chr8 | 19819029 | 19819149; |
| Probe 336 | chr8 | 19819134 | 19819254; |
| Probe 337 | chr8 | 19819239 | 19819359; |
| Probe 338 | chr8 | 19819344 | 19819464; |
| Probe 339 | chr8 | 19819449 | 19819569; |
| Probe 340 | chr8 | 19819554 | 19819674; |
| Probe 341 | chr8 | 19819659 | 19819779; |
| Probe 342 | chr8 | 19819764 | 19819884; |
| Probe 343 | chr8 | 19819869 | 19819989; |
| Probe 344 | chr8 | 19819974 | 19820094; |
| Probe 345 | chr8 | 19820079 | 19820199; |
| Probe 346 | chr8 | 19820184 | 19820304; |
| Probe 347 | chr8 | 19820289 | 19820409; |
| Probe 348 | chr8 | 19820394 | 19820514; |
| Probe 349 | chr8 | 19820499 | 19820619; |
| Probe 350 | chr8 | 19820604 | 19820724; |
| Probe 351 | chr8 | 19820919 | 19821039; |
| Probe 352 | chr8 | 19821024 | 19821144; |
| Probe 353 | chr8 | 19821129 | 19821249; |
| Probe 354 | chr8 | 19821234 | 19821354; |
| Probe 355 | chr8 | 19821339 | 19821459; |
| Probe 356 | chr8 | 19821759 | 19821879; |
| Probe 357 | chr8 | 19821864 | 19821984; |
| Probe 358 | chr8 | 19821969 | 19822089; |
| Probe 359 | chr8 | 19822284 | 19822404; |
| Probe 360 | chr8 | 19822389 | 19822509; |
| Probe 361 | chr8 | 19822494 | 19822614; |
| Probe 362 | chr8 | 19822599 | 19822719; |
| Probe 363 | chr8 | 19822704 | 19822824; |
| Probe 364 | chr8 | 19822809 | 19822929; |
| Probe 365 | chr8 | 19822914 | 19823034; |
| Probe 366 | chr8 | 19823019 | 19823139; |
| Probe 367 | chr8 | 19823124 | 19823244; |
| Probe 368 | chr8 | 19823229 | 19823349; |
| Probe 369 | chr8 | 19823334 | 19823454; |
| Probe 370 | chr8 | 19823439 | 19823559; |
| Probe 371 | chr8 | 19823544 | 19823664; |
| Probe 372 | chr8 | 19823649 | 19823769; |
| Probe 373 | chr8 | 19823754 | 19823874; |
| Probe 374 | chr8 | 19823859 | 19823979; |
| Probe 375 | chr8 | 19823964 | 19824084; |
| Probe 376 | chr8 | 19824069 | 19824189; |
| Probe 377 | chr8 | 19824174 | 19824294; |
| Probe 378 | chr8 | 19824279 | 19824399; |
| Probe 379 | chr8 | 19824384 | 19824504; |
| Probe 380 | chr8 | 19824489 | 19824609; |
| Probe 381 | chr8 | 19824594 | 19824714; |
| Probe 382 | chr8 | 19824699 | 19824819; |
| Probe 383 | chr8 | 144294573 | 144294693; |
| Probe 384 | chr8 | 144294678 | 144294798; |
| Probe 385 | chr8 | 144294783 | 144294903; |
| Probe 386 | chr8 | 144294888 | 144295008; |
| Probe 387 | chr8 | 144294993 | 144295113; |
| Probe 388 | chr8 | 144295098 | 144295218; |
| Probe 389 | chr8 | 144295203 | 144295323; |
| Probe 390 | chr8 | 144295308 | 144295428; |
| Probe 391 | chr8 | 144295413 | 144295533; |
| Probe 392 | chr8 | 144295518 | 144295638; |
| Probe 393 | chr8 | 144295623 | 144295743; |
| Probe 394 | chr8 | 144295728 | 144295848; |
| Probe 395 | chr8 | 144295833 | 144295953; |
| Probe 396 | chr8 | 144295938 | 144296058; |
| Probe 397 | chr8 | 144296043 | 144296163; |
| Probe 398 | chr8 | 144296148 | 144296268; |
| Probe 399 | chr8 | 144296253 | 144296373; |
| Probe 400 | chr8 | 144296358 | 144296478; |
| Probe 401 | chr8 | 144296463 | 144296583; |
| Probe 402 | chr8 | 144296568 | 144296688; |
| Probe 403 | chr8 | 144296673 | 144296793; |
| Probe 404 | chr8 | 144296778 | 144296898; |
| Probe 405 | chr8 | 144296883 | 144297003; |
| Probe 406 | chr8 | 144296988 | 144297108; |
| Probe 407 | chr8 | 144297093 | 144297213; |
| Probe 408 | chr8 | 144297198 | 144297318; |
| Probe 409 | chr8 | 144297303 | 144297423; |
| Probe 410 | chr8 | 144297408 | 144297528; |
| Probe 411 | chr8 | 144297513 | 144297633; |
| Probe 412 | chr8 | 144297618 | 144297738; |
| Probe 413 | chr8 | 144297723 | 144297843; |
| Probe 414 | chr8 | 144297828 | 144297948; |
| Probe 415 | chr8 | 144297933 | 144298053; |
| Probe 416 | chr8 | 144298038 | 144298158; |
| Probe 417 | chr8 | 144298143 | 144298263; |
| Probe 418 | chr8 | 144298248 | 144298368; |
| Probe 419 | chr8 | 144298353 | 144298473; |
| Probe 420 | chr8 | 144298458 | 144298578; |
| Probe 421 | chr8 | 144298563 | 144298683; |
| Probe 422 | chr8 | 144298668 | 144298788; |
| Probe 423 | chr8 | 144298773 | 144298893; |
| Probe 424 | chr8 | 144298878 | 144298998; |
| Probe 425 | chr8 | 144298983 | 144299103; |

The reference genome is Hg19.

The capture probes provided in the embodiments of the present application are designed to cover the exons and hotspot intron regions of 6 genes closely related to FCS (*LPL, APOC2, LMF1, GPIHBP1, APOA5* and *GPD1*). It should be understood that, according to actual needs, some of the probe sets may be selected to detect some of the target genes, and they may be, for example, any one, two, three, four, or five probe sets selected from the probe sets 1 to 6. In order to meet the detection requirements of certain regions of the target gene, a corresponding number of probes may be selected from the corresponding probe set, for example, selecting 1 probe, 2 probes, 3 probes... 24 probes, 25 probes, and 26 probes from the probe set 1.
Optionally, the capture probes comprise a plurality of probe sets selected from the probe set 1 to probe set 6;
The probe set 1 comprises a plurality of probes selected from probes 1 to 26;
The probe set 2 comprises a plurality of probes selected from the probe 27 to the probe 64;
The probe set 3 comprises a plurality of probes selected from the probe 65 to the probe 112;
The probe set 4 comprises a plurality of probes selected from the probe 113 to the probe 139;
The probe set 5 comprises a plurality of probes selected from the probe 140 to the probe 382;
The probe set 6 comprises a plurality of probes selected from the probe 383 to the probe 425.
Further optionally, the capture probes comprise the probe set 1 to the probe set 6;
The probe set 1 comprises probes 1 to 26;
The probe set 2 comprises the probe 27 to the probe 64;
The probe set 3 comprises the probe 65 to the probe 112;
The probe set 4 comprises the probe 113 to the probe 139;
The probe set 5 comprises the probe 140 to the probe 382;
The probe set 6 comprises the probe 383 to the probe 425.

### A second aspect of the embodiments of the present application

The embodiments of the present application provide use of the capture probes in preparing a familial chylomicronemia syndrome detection kit.

### A third aspect of the embodiments of the present application

The embodiments of the present application provide a familial chylomicronemia syndrome detection kit comprising the capture probes.

Optionally, the familial chylomicronemia syndrome detection kit further includes one or more of a nucleic acid extraction reagent, a DNA library construction reagent, a sample and library quantification reagent, a fragment quality control reagent, a hybridization capture reagent, a nucleic acid purification reagent, a target gene fragment amplification reagent, and a sequencing reagent.

### A fourth aspect of the embodiments of the present application

The embodiments of the present application provide use of the capture probes in constructing a detection library for gene mutations related to familial chylomicronemia syndrome.

### A fifth aspect of the embodiments of the present application

The embodiments of the present application provide use of the detection kit in constructing a detection library for gene mutations related to familial chylomicronemia syndrome.

### A sixth aspect of the embodiments of the present application

The embodiments of the present application provide a method for detecting familial chylomicronemia syndrome-related gene mutations, comprising using the capture probes or the detection kit to capture target gene fragments containing gene mutations related to familial chylomicronemia syndrome, and detecting the captured fragments.

Optionally, sequencing is used to detect the captured fragments.

In the following specific embodiments, measurement parameters involving raw material components may, unless otherwise specifically stated, have slight deviations within the weighing accuracy range. For the temperature and time parameters involved, deviations caused by instrument testing accuracy or operational accuracy are allowable.

The reagents used in the detection process of this embodiment is listed as follows:

**Table 1**

| No. | Function | Name of reagent consumable | Brand | Specification |
|---|---|---|---|---|
| 1 | Nucleic acid extraction | QIAamp DNA Mini Kit(250) | QIAGEN | 51306 |
| 2 | Nucleic acid quantification | Qubit^{™}dsDNA HS Assay Kit | ThermoFisher | Q32854 |
| 3 | Specifically hydrolyzing cytosine (C) or uracil (U) residues of RNA | RNase A (17,500 U) | QIAGEN | 19101 |
| 4 | Constructing DNA libraries | VAHTS Universal DNA Library Prep Kit for Illumina V3 | Vazyme | ND607-02 |
| 5 | DNA amplification | KAPA HiFi HotStart ReadyMix | Kapa Biosystems | KK2602 |
| 6 | Library construction adapter | NEXTflex^{®}Dual-Indexed DNA Barcodes(1-96) | Bioo Scientific | NOVA-514160 |
| 7 | Library construction adapter | NEXTflex^{®}Dual-Indexed DNA Barcodes (97-192) | Bioo Scientific | NOVA-514161 |
| 8 | Purifying nucleic acid | Agencourt AMPure XP 450mL Kit | Beckman Coulter | A63882 |
| 9 | Blocking sample adapter sequences | xGen^{®}Universal Blockers-TS Mix,4x96rxn | Integrated DNA Technologies | 1075476 |
| 10 | Binding and capturing nucleic acids complementary to the target sequence | FCS_Probes (Table 4) | Nanodigmbio | customized |
| 11 | Targeted capture hybridization and washing | xGen^{®} Hybridization and Wash Kit | Integrated DNA Technologies | 1080584 |
| 12 | Complementarily paired P5 sequence | Illumina P5 Primer | customized | customized |
| 13 | Complementarily paired P7 sequence | Illumina P7 Primer | customized | customized |
| 14 | DNA molecular weight determination and quantitative analysis | DNA High Sensitivity Reagent Kit | PerkinElmer | CLS760672 |
| 15 | Microfluidic chip | DNA Extended Range Chip for use with GX Touch/GXII Touch HT | PerkinElmer | 760517 |
| 16 | Buffer component | TWEEN^{®}20 | SigmaAldrich | P7949 |
| 17 | Buffer component | 1M Tris pH 7.0 | ThermoFisher | AM9851 |
| 18 | Buffer component | Sodium hydroxide solution | Fluka Analytical | 319511-500ML |
| 19 | Buffer component | Sodium hypochlorite solution | SigmaAldrich | 239305 |
| 20 | Calibration quality control | PhiX Control v3 | Illumina | FC-110-3001 |
| 21 | Cluster generation and sequencing | NovaSeq 6000 v2 kit (300cycles) | Illumina | FC-404-2004 |

The equipment used in the detection process of this embodiment is listed as follows:

**Table 2**

| No. | Function | Equipment name | Factory number | Model |
|---|---|---|---|---|
| 1 | Vortex oscillation | Vortex-Genie 2 | Scientific Industries | SI-0246 |
| 2 | High-speed centrifugation | Heraeus Pico 21 Centrifuge, Ventilated | ThermoFisher | 75002415 |
| 3 | Dry constant temperature | GL-150B Dry Thermostat | Kylin-Bell | GL-150B |
| 4 | Instant rapid centrifugation | TGear Mini centrifuge (OSE-MC8) | TIANGEN BIOTECH (BEIJING) | OSE-MC8 |
| 5 | Nucleic acid quantification | Qubit 3 Fluorometer | ThermoFisher | Q33216 |
| 6 | Nucleic acid quantitative analysis and purity assessment | NanoDrop^{™} 2000 Spectrophotometer | ThermoFisher | ND-2000 |
| 7 | PCR nucleic acid amplification | GeneAmp^{®} PCR System 9700, 96-Well Aluminum | ThermoFisher | 4314879 |
| 8 | Electrophoresis power supply | PowerPac^{™} Basic Power Supply | Bio-Rad | 1645050 |
| 9 | Gel imaging analysis | Tanon 2500 Gel Imaging System | Tanon Science & Technology | Tanon-2500 |
| 10 | Ultrasonic fragmentation of samples | M220 Focused-ultrasonicator^{™} | Covaris | 500295 |
| 11 | PCR nucleic acid amplification | Veriti^{™} 96-Well Thermal Cycler | ThermoFisher | 4375786 |
| 12 | Vacuum concentration of nucleic acid | Savant^{™} DNA SpeedVac^{™} Concentrator | ThermoFisher | DNA120-230 |
| 13 | Performing electrophoretic separation as well as qualitative and quantitative analysis of nucleic acid | LabChip GX Touch HT Nucleic Acid Analyzer | PerkinElmer | CLS137031 |
| 14 | Aspiration, discharge | GL-802 Compact Desktop Vacuum Pump | Kylin-Bell | GL-802 |
| 15 | Microplate centrifugation | MPS 1000 Mini Plate Spinner Centrifuge | Labnet | C1000 |
| 16 | Preparation of pure water and ultrapure water | Direct-Q^{®} 3 UV Water Purification System | MerckMillipore | ZRQSVP3CN |
| 17 | High-throughput sequencing | NovaSeq^{™} 6000 Dx | Illumina | A00335 |

### Example 1

In this example, the capture sequencing detection process for 6 genes related to FCS is divided into four steps: sample preparation, library construction, target region capture, and sequencing.

### 1. Panel design

In this embodiment, the region covered by the target capture probe panel for detecting 6 genes related to FCS, namely *LPL, APOC2, LMF1, GPIHBP1, APOA5* and *GPD1,* is 18.6 kb, covering all exons and hotspot intron regions of the 6 genes closely related to FCS (Table 3). The high-density probe design ensures that each target region will be covered by at least two probes (see Table 4).

**Table 3. Probe coverage ranges for 6 FCS-related genes**

| Gene | Coverage |
|---|---|
| *APOC2* | Full exon+500bp upstream, 49.1% intron. |
| *LPL* | Full exon+500bp upstream, 89.3% intron. |
| *GPIHBP1* | Full gene+500bp upstream. |
| *APOA5* | Full exon+500bp upstream. |
| *GPD1* | Full exon+500bp upstream. |
| *LMF1* | Full exon+500bp upstream. |

**Table 4. Capture probes**

| **Number** | **Chr** | **Start** | **End** | **Number** | **Chr** | **Start** | **End** |
|---|---|---|---|---|---|---|---|
| 1 | chr11 | 116660063 | 116660183 | 14 | chr11 | 116661428 | 116661548 |
| 2 | chr11 | 116660168 | 116660288 | 15 | chr11 | 116661533 | 116661653 |
| 3 | chr11 | 116660273 | 116660393 | 16 | chr11 | 116661638 | 116661758 |
| 4 | chr11 | 116660378 | 116660498 | 17 | chr11 | 116661743 | 116661863 |
| 5 | chr11 | 116660483 | 116660603 | 18 | chr11 | 116662282 | 116662402 |
| 6 | chr11 | 116660588 | 116660708 | 19 | chr11 | 116662387 | 116662507 |
| 7 | chr11 | 116660693 | 116660813 | 20 | chr11 | 116662508 | 116662628 |
| 8 | chr11 | 116660798 | 116660918 | 21 | chr11 | 116663076 | 116663196 |
| 9 | chr11 | 116660903 | 116661023 | 22 | chr11 | 116663181 | 116663301 |
| 10 | chr11 | 116661008 | 116661128 | 23 | chr11 | 116663286 | 116663406 |
| 11 | chr11 | 116661113 | 116661233 | 24 | chr11 | 116663391 | 116663511 |
| 12 | chr11 | 116661218 | 116661338 | 25 | chr11 | 116663496 | 116663616 |
| 13 | chr11 | 116661323 | 116661443 | 26 | chr11 | 116663601 | 116663721 |
| 27 | chr12 | 50497271 | 50497391 | 46 | chr12 | 50503186 | 50503306 |
| 28 | chr12 | 50497376 | 50497496 | 47 | chr12 | 50503291 | 50503411 |
| 29 | chr12 | 50497481 | 50497601 | 48 | chr12 | 50503396 | 50503516 |
| 30 | chr12 | 50497586 | 50497706 | 49 | chr12 | 50503501 | 50503621 |
| 31 | chr12 | 50497691 | 50497811 | 50 | chr12 | 50503606 | 50503726 |
| 32 | chr12 | 50497796 | 50497916 | 51 | chr12 | 50503711 | 50503831 |
| 33 | chr12 | 50498337 | 50498457 | 52 | chr12 | 50503816 | 50503936 |
| 34 | chr12 | 50498442 | 50498562 | 53 | chr12 | 50503921 | 50504041 |
| 35 | chr12 | 50499311 | 50499431 | 54 | chr12 | 50504026 | 50504146 |
| 36 | chr12 | 50499416 | 50499536 | 55 | chr12 | 50504131 | 50504251 |
| 37 | chr12 | 50500051 | 50500171 | 56 | chr12 | 50504236 | 50504356 |
| 38 | chr12 | 50500156 | 50500276 | 57 | chr12 | 50504341 | 50504461 |
| 39 | chr12 | 50500568 | 50500688 | 58 | chr12 | 50504446 | 50504566 |
| 40 | chr12 | 50500673 | 50500793 | 59 | chr12 | 50504551 | 50504671 |
| 41 | chr12 | 50501330 | 50501450 | 60 | chr12 | 50504656 | 50504776 |
| 42 | chr12 | 50501435 | 50501555 | 61 | chr12 | 50504761 | 50504881 |
| 43 | chr12 | 50501540 | 50501660 | 62 | chr12 | 50504866 | 50504986 |
| 44 | chr12 | 50501787 | 50501907 | 63 | chr12 | 50504971 | 50505091 |
| 45 | chr12 | 50501892 | 50502012 | 64 | chr12 | 50505076 | 50505196 |
| 65 | chr16 | 903614 | 903734 | 89 | chr16 | 960911 | 961031 |
| 66 | chr16 | 903719 | 903839 | 90 | chr16 | 961016 | 961136 |
| 67 | chr16 | 903824 | 903944 | 91 | chr16 | 981622 | 981742 |
| 68 | chr16 | 903929 | 904049 | 92 | chr16 | 981727 | 981847 |
| 69 | chr16 | 904034 | 904154 | 93 | chr16 | 983971 | 984091 |
| 70 | chr16 | 904139 | 904259 | 94 | chr16 | 984076 | 984196 |
| 71 | chr16 | 904244 | 904364 | 95 | chr16 | 984170 | 984290 |
| 72 | chr16 | 904349 | 904469 | 96 | chr16 | 1004337 | 1004457 |
| 73 | chr16 | 904454 | 904574 | 97 | chr16 | 1004442 | 1004562 |
| 74 | chr16 | 904559 | 904679 | 98 | chr16 | 1004547 | 1004667 |
| 75 | chr16 | 904664 | 904784 | 99 | chr16 | 1004652 | 1004772 |
| 76 | chr16 | 905087 | 905207 | 100 | chr16 | 1020768 | 1020888 |
| 77 | chr16 | 905192 | 905312 | 101 | chr16 | 1020873 | 1020993 |
| 78 | chr16 | 918924 | 919044 | 102 | chr16 | 1020978 | 1021098 |
| 79 | chr16 | 919029 | 919149 | 103 | chr16 | 1031125 | 1031245 |
| 80 | chr16 | 919863 | 919983 | 104 | chr16 | 1031230 | 1031350 |
| 81 | chr16 | 919968 | 920088 | 105 | chr16 | 1031335 | 1031455 |
| 82 | chr16 | 920709 | 920829 | 106 | chr16 | 1031440 | 1031560 |
| 83 | chr16 | 920814 | 920934 | 107 | chr16 | 1031545 | 1031665 |
| 84 | chr16 | 921141 | 921261 | 108 | chr16 | 1031650 | 1031770 |
| 85 | chr16 | 921246 | 921366 | 109 | chr16 | 1031755 | 1031875 |
| 86 | chr16 | 929550 | 929670 | 110 | chr16 | 1031860 | 1031980 |
| 87 | chr16 | 929655 | 929775 | 111 | chr16 | 1031965 | 1032085 |
| 88 | chr16 | 942987 | 943107 | 112 | chr16 | 1032070 | 1032190 |
| 113 | chr19 | 45448788 | 45448908 | 126 | chr19 | 45450678 | 45450798 |
| 114 | chr19 | 45448893 | 45449013 | 127 | chr19 | 45451518 | 45451638 |
| 115 | chr19 | 45448998 | 45449118 | 128 | chr19 | 45451623 | 45451743 |
| 116 | chr19 | 45449103 | 45449223 | 129 | chr19 | 45451728 | 45451848 |
| 117 | chr19 | 45449208 | 45449328 | 130 | chr19 | 45451833 | 45451953 |
| 118 | chr19 | 45449313 | 45449433 | 131 | chr19 | 45451938 | 45452058 |
| 119 | chr19 | 45449418 | 45449538 | 132 | chr19 | 45452043 | 45452163 |
| 120 | chr19 | 45449628 | 45449748 | 133 | chr19 | 45452148 | 45452268 |
| 121 | chr19 | 45449733 | 45449853 | 134 | chr19 | 45452253 | 45452373 |
| 122 | chr19 | 45450048 | 45450168 | 135 | chr19 | 45452358 | 45452478 |
| 123 | chr19 | 45450363 | 45450483 | 136 | chr19 | 45452463 | 45452583 |
| 124 | chr19 | 45450468 | 45450588 | 137 | chr19 | 45452568 | 45452688 |
| 125 | chr19 | 45450573 | 45450693 | 138 | chr19 | 45452673 | 45452793 |
| 140 | chr8 | 19796244 | 19796364 | 139 | chr19 | 45452778 | 45452898 |
| 141 | chr8 | 19796349 | 19796469 | 262 | chr8 | 19810314 | 19810434 |
| 142 | chr8 | 19796454 | 19796574 | 263 | chr8 | 19810419 | 19810539 |
| 143 | chr8 | 19796559 | 19796679 | 264 | chr8 | 19810524 | 19810644 |
| 144 | chr8 | 19796664 | 19796784 | 265 | chr8 | 19810629 | 19810749 |
| 145 | chr8 | 19796769 | 19796889 | 266 | chr8 | 19810734 | 19810854 |
| 146 | chr8 | 19796874 | 19796994 | 267 | chr8 | 19810839 | 19810959 |
| 147 | chr8 | 19796979 | 19797099 | 268 | chr8 | 19810944 | 19811064 |
| 148 | chr8 | 19797084 | 19797204 | 269 | chr8 | 19811049 | 19811169 |
| 149 | chr8 | 19797189 | 19797309 | 270 | chr8 | 19811154 | 19811274 |
| 150 | chr8 | 19797294 | 19797414 | 271 | chr8 | 19811259 | 19811379 |
| 151 | chr8 | 19797399 | 19797519 | 272 | chr8 | 19811364 | 19811484 |
| 152 | chr8 | 19797504 | 19797624 | 273 | chr8 | 19811469 | 19811589 |
| 153 | chr8 | 19797609 | 19797729 | 274 | chr8 | 19811574 | 19811694 |
| 154 | chr8 | 19797714 | 19797834 | 275 | chr8 | 19811679 | 19811799 |
| 155 | chr8 | 19797819 | 19797939 | 276 | chr8 | 19811784 | 19811904 |
| 156 | chr8 | 19797924 | 19798044 | 277 | chr8 | 19811889 | 19812009 |
| 157 | chr8 | 19798029 | 19798149 | 278 | chr8 | 19811994 | 19812114 |
| 158 | chr8 | 19798134 | 19798254 | 279 | chr8 | 19812099 | 19812219 |
| 159 | chr8 | 19798239 | 19798359 | 280 | chr8 | 19812204 | 19812324 |
| 160 | chr8 | 19798344 | 19798464 | 281 | chr8 | 19812309 | 19812429 |
| 161 | chr8 | 19798449 | 19798569 | 282 | chr8 | 19812519 | 19812639 |
| 162 | chr8 | 19798554 | 19798674 | 283 | chr8 | 19812624 | 19812744 |
| 163 | chr8 | 19798659 | 19798779 | 284 | chr8 | 19812729 | 19812849 |
| 164 | chr8 | 19798764 | 19798884 | 285 | chr8 | 19812834 | 19812954 |
| 165 | chr8 | 19798869 | 19798989 | 286 | chr8 | 19812939 | 19813059 |
| 166 | chr8 | 19798974 | 19799094 | 287 | chr8 | 19813044 | 19813164 |
| 167 | chr8 | 19799079 | 19799199 | 288 | chr8 | 19813149 | 19813269 |
| 168 | chr8 | 19799184 | 19799304 | 289 | chr8 | 19813254 | 19813374 |
| 169 | chr8 | 19799289 | 19799409 | 290 | chr8 | 19813359 | 19813479 |
| 170 | chr8 | 19799394 | 19799514 | 291 | chr8 | 19813464 | 19813584 |
| 171 | chr8 | 19799499 | 19799619 | 292 | chr8 | 19813569 | 19813689 |
| 172 | chr8 | 19799604 | 19799724 | 293 | chr8 | 19813674 | 19813794 |
| 173 | chr8 | 19799709 | 19799829 | 294 | chr8 | 19813779 | 19813899 |
| 174 | chr8 | 19799814 | 19799934 | 295 | chr8 | 19813884 | 19814004 |
| 175 | chr8 | 19799919 | 19800039 | 296 | chr8 | 19814094 | 19814214 |
| 176 | chr8 | 19800024 | 19800144 | 297 | chr8 | 19814199 | 19814319 |
| 177 | chr8 | 19800129 | 19800249 | 298 | chr8 | 19814304 | 19814424 |
| 178 | chr8 | 19800234 | 19800354 | 299 | chr8 | 19814619 | 19814739 |
| 179 | chr8 | 19800339 | 19800459 | 300 | chr8 | 19814724 | 19814844 |
| 180 | chr8 | 19800444 | 19800564 | 301 | chr8 | 19814829 | 19814949 |
| 181 | chr8 | 19800549 | 19800669 | 302 | chr8 | 19814934 | 19815054 |
| 182 | chr8 | 19800654 | 19800774 | 303 | chr8 | 19815039 | 19815159 |
| 183 | chr8 | 19800759 | 19800879 | 304 | chr8 | 19815144 | 19815264 |
| 184 | chr8 | 19800864 | 19800984 | 305 | chr8 | 19815249 | 19815369 |
| 185 | chr8 | 19800969 | 19801089 | 306 | chr8 | 19815354 | 19815474 |
| 186 | chr8 | 19801074 | 19801194 | 307 | chr8 | 19815459 | 19815579 |
| 187 | chr8 | 19801179 | 19801299 | 308 | chr8 | 19815774 | 19815894 |
| 188 | chr8 | 19801284 | 19801404 | 309 | chr8 | 19815879 | 19815999 |
| 189 | chr8 | 19801704 | 19801824 | 310 | chr8 | 19815984 | 19816104 |
| 190 | chr8 | 19801809 | 19801929 | 311 | chr8 | 19816194 | 19816314 |
| 191 | chr8 | 19801914 | 19802034 | 312 | chr8 | 19816299 | 19816419 |
| 192 | chr8 | 19802019 | 19802139 | 313 | chr8 | 19816404 | 19816524 |
| 193 | chr8 | 19802124 | 19802244 | 314 | chr8 | 19816509 | 19816629 |
| 194 | chr8 | 19802229 | 19802349 | 315 | chr8 | 19816614 | 19816734 |
| 195 | chr8 | 19802334 | 19802454 | 316 | chr8 | 19816719 | 19816839 |
| 196 | chr8 | 19802439 | 19802559 | 317 | chr8 | 19816824 | 19816944 |
| 197 | chr8 | 19802544 | 19802664 | 318 | chr8 | 19816929 | 19817049 |
| 198 | chr8 | 19802649 | 19802769 | 319 | chr8 | 19817034 | 19817154 |
| 199 | chr8 | 19802754 | 19802874 | 320 | chr8 | 19817139 | 19817259 |
| 200 | chr8 | 19802859 | 19802979 | 321 | chr8 | 19817559 | 19817679 |
| 201 | chr8 | 19802964 | 19803084 | 322 | chr8 | 19817664 | 19817784 |
| 202 | chr8 | 19803069 | 19803189 | 323 | chr8 | 19817769 | 19817889 |
| 203 | chr8 | 19803174 | 19803294 | 324 | chr8 | 19817874 | 19817994 |
| 204 | chr8 | 19803279 | 19803399 | 325 | chr8 | 19817979 | 19818099 |
| 205 | chr8 | 19803384 | 19803504 | 326 | chr8 | 19818084 | 19818204 |
| 206 | chr8 | 19803489 | 19803609 | 327 | chr8 | 19818189 | 19818309 |
| 207 | chr8 | 19803594 | 19803714 | 328 | chr8 | 19818294 | 19818414 |
| 208 | chr8 | 19803699 | 19803819 | 329 | chr8 | 19818399 | 19818519 |
| 209 | chr8 | 19803804 | 19803924 | 330 | chr8 | 19818504 | 19818624 |
| 210 | chr8 | 19803909 | 19804029 | 331 | chr8 | 19818609 | 19818729 |
| 211 | chr8 | 19804014 | 19804134 | 332 | chr8 | 19818714 | 19818834 |
| 212 | chr8 | 19804119 | 19804239 | 333 | chr8 | 19818819 | 19818939 |
| 213 | chr8 | 19804224 | 19804344 | 334 | chr8 | 19818924 | 19819044 |
| 214 | chr8 | 19804329 | 19804449 | 335 | chr8 | 19819029 | 19819149 |
| 215 | chr8 | 19804434 | 19804554 | 336 | chr8 | 19819134 | 19819254 |
| 216 | chr8 | 19804539 | 19804659 | 337 | chr8 | 19819239 | 19819359 |
| 217 | chr8 | 19804644 | 19804764 | 338 | chr8 | 19819344 | 19819464 |
| 218 | chr8 | 19805064 | 19805184 | 339 | chr8 | 19819449 | 19819569 |
| 219 | chr8 | 19805169 | 19805289 | 340 | chr8 | 19819554 | 19819674 |
| 220 | chr8 | 19805274 | 19805394 | 341 | chr8 | 19819659 | 19819779 |
| 221 | chr8 | 19805379 | 19805499 | 342 | chr8 | 19819764 | 19819884 |
| 222 | chr8 | 19805484 | 19805604 | 343 | chr8 | 19819869 | 19819989 |
| 223 | chr8 | 19805589 | 19805709 | 344 | chr8 | 19819974 | 19820094 |
| 224 | chr8 | 19805694 | 19805814 | 345 | chr8 | 19820079 | 19820199 |
| 225 | chr8 | 19805799 | 19805919 | 346 | chr8 | 19820184 | 19820304 |
| 226 | chr8 | 19805904 | 19806024 | 347 | chr8 | 19820289 | 19820409 |
| 227 | chr8 | 19806009 | 19806129 | 348 | chr8 | 19820394 | 19820514 |
| 228 | chr8 | 19806114 | 19806234 | 349 | chr8 | 19820499 | 19820619 |
| 229 | chr8 | 19806219 | 19806339 | 350 | chr8 | 19820604 | 19820724 |
| 230 | chr8 | 19806324 | 19806444 | 351 | chr8 | 19820919 | 19821039 |
| 231 | chr8 | 19806429 | 19806549 | 352 | chr8 | 19821024 | 19821144 |
| 232 | chr8 | 19806534 | 19806654 | 353 | chr8 | 19821129 | 19821249 |
| 233 | chr8 | 19806639 | 19806759 | 354 | chr8 | 19821234 | 19821354 |
| 234 | chr8 | 19806744 | 19806864 | 355 | chr8 | 19821339 | 19821459 |
| 235 | chr8 | 19806849 | 19806969 | 356 | chr8 | 19821759 | 19821879 |
| 236 | chr8 | 19807269 | 19807389 | 357 | chr8 | 19821864 | 19821984 |
| 237 | chr8 | 19807374 | 19807494 | 358 | chr8 | 19821969 | 19822089 |
| 238 | chr8 | 19807479 | 19807599 | 359 | chr8 | 19822284 | 19822404 |
| 239 | chr8 | 19807584 | 19807704 | 360 | chr8 | 19822389 | 19822509 |
| 240 | chr8 | 19807689 | 19807809 | 361 | chr8 | 19822494 | 19822614 |
| 241 | chr8 | 19807794 | 19807914 | 362 | chr8 | 19822599 | 19822719 |
| 242 | chr8 | 19807899 | 19808019 | 363 | chr8 | 19822704 | 19822824 |
| 243 | chr8 | 19808319 | 19808439 | 364 | chr8 | 19822809 | 19822929 |
| 244 | chr8 | 19808424 | 19808544 | 365 | chr8 | 19822914 | 19823034 |
| 245 | chr8 | 19808529 | 19808649 | 366 | chr8 | 19823019 | 19823139 |
| 246 | chr8 | 19808634 | 19808754 | 367 | chr8 | 19823124 | 19823244 |
| 247 | chr8 | 19808739 | 19808859 | 368 | chr8 | 19823229 | 19823349 |
| 248 | chr8 | 19808844 | 19808964 | 369 | chr8 | 19823334 | 19823454 |
| 249 | chr8 | 19808949 | 19809069 | 370 | chr8 | 19823439 | 19823559 |
| 250 | chr8 | 19809054 | 19809174 | 371 | chr8 | 19823544 | 19823664 |
| 251 | chr8 | 19809159 | 19809279 | 372 | chr8 | 19823649 | 19823769 |
| 252 | chr8 | 19809264 | 19809384 | 373 | chr8 | 19823754 | 19823874 |
| 253 | chr8 | 19809369 | 19809489 | 374 | chr8 | 19823859 | 19823979 |
| 254 | chr8 | 19809474 | 19809594 | 375 | chr8 | 19823964 | 19824084 |
| 255 | chr8 | 19809579 | 19809699 | 376 | chr8 | 19824069 | 19824189 |
| 256 | chr8 | 19809684 | 19809804 | 377 | chr8 | 19824174 | 19824294 |
| 257 | chr8 | 19809789 | 19809909 | 378 | chr8 | 19824279 | 19824399 |
| 258 | chr8 | 19809894 | 19810014 | 379 | chr8 | 19824384 | 19824504 |
| 259 | chr8 | 19809999 | 19810119 | 380 | chr8 | 19824489 | 19824609 |
| 260 | chr8 | 19810104 | 19810224 | 381 | chr8 | 19824594 | 19824714 |
| 261 | chr8 | 19810209 | 19810329 | 382 | chr8 | 19824699 | 19824819 |
| 383 | chr8 | 144294573 | 144294693 | 405 | chr8 | 144296883 | 144297003 |
| 384 | chr8 | 144294678 | 144294798 | 406 | chr8 | 144296988 | 144297108 |
| 385 | chr8 | 144294783 | 144294903 | 407 | chr8 | 144297093 | 144297213 |
| 386 | chr8 | 144294888 | 144295008 | 408 | chr8 | 144297198 | 144297318 |
| 387 | chr8 | 144294993 | 144295113 | 409 | chr8 | 144297303 | 144297423 |
| 388 | chr8 | 144295098 | 144295218 | 410 | chr8 | 144297408 | 144297528 |
| 389 | chr8 | 144295203 | 144295323 | 411 | chr8 | 144297513 | 144297633 |
| 390 | chr8 | 144295308 | 144295428 | 412 | chr8 | 144297618 | 144297738 |
| 391 | chr8 | 144295413 | 144295533 | 413 | chr8 | 144297723 | 144297843 |
| 392 | chr8 | 144295518 | 144295638 | 414 | chr8 | 144297828 | 144297948 |
| 393 | chr8 | 144295623 | 144295743 | 415 | chr8 | 144297933 | 144298053 |
| 394 | chr8 | 144295728 | 144295848 | 416 | chr8 | 144298038 | 144298158 |
| 395 | chr8 | 144295833 | 144295953 | 417 | chr8 | 144298143 | 144298263 |
| 396 | chr8 | 144295938 | 144296058 | 418 | chr8 | 144298248 | 144298368 |
| 397 | chr8 | 144296043 | 144296163 | 419 | chr8 | 144298353 | 144298473 |
| 398 | chr8 | 144296148 | 144296268 | 420 | chr8 | 144298458 | 144298578 |
| 399 | chr8 | 144296253 | 144296373 | 421 | chr8 | 144298563 | 144298683 |
| 400 | chr8 | 144296358 | 144296478 | 422 | chr8 | 144298668 | 144298788 |
| 401 | chr8 | 144296463 | 144296583 | 423 | chr8 | 144298773 | 144298893 |
| 402 | chr8 | 144296568 | 144296688 | 424 | chr8 | 144298878 | 144298998 |
| 403 | chr8 | 144296673 | 144296793 | 425 | chr8 | 144298983 | 144299103 |
| 404 | chr8 | 144296778 | 144296898 | | | | |

In this example, target capture probes covered all exons and hotspot intronic regions of the above six genes to ensure accurate and efficient detection of FCS-related gene mutations and to provide a basis for the clinical diagnosis and medication guidance of FCS. The nucleotide sequences of the probes 1 to 425 are shown in SEQ ID NOs. 1 to 425.

### 2. Detection experiment design

The detection workflow includes four main steps: sample preparation, library construction, target region enrichment, and sequencing. First, about 300 ng of gDNA was taken and fragmented into DNA fragments of about 200 bp by using a Covaris ultrasonic fragmenter (see step 2.1), library preparation was carried out by using an Illumina V3 VAHTS Universal DNA Library Prep Kit (Vazyme ND607-02), and the library was quantified by using a Qubit^{®} dsDNA HS Assay Kit (Thermo Fisher) (see step 2.2). An xGen hybridization and capture kit (Integrated DNA Technologies, 1080584) was used to perform FCS probe capture on the library, and after capture, library quality control (QC) was performed using a Qubit fluorometer (Invitrogen) and LabChip GX Touch HT (Perkin Elmer). Finally, sequencing was carried out on a NovaSeq6000 platform (Illumina) (2×150 bp) (see step 2.4).

The following is a detailed description of each experimental step:

### 2.1 Sample preparation

Ten mutant gDNA samples containing known variants in six genes (*LPL*, *APOC2*, *LMF1*, *GPIHBP1*, *APOA5*, and *GPD1*) (for specific gene variant types, see attached Table 5), and 10 wild-type gDNA samples not containing any variant type of the above 6 genes (Table 5) were selected. For each gDNA sample, about 300 ng was taken, IDTE buffer (10 mM Tris, 0.1 mM EDTA) was added to make up the volume to 50µL, and the Covaris ultrasonic fragmenter was set according to the parameters in Table 6 below to fragment the gDNA samples for 180 s.

**Table 5. Sample information**

| Sample number | Gene | Variant information | Sample number | Genotype |
|---|---|---|---|---|
| PN1 | LPL | c.249 + 1G > A | N1 | Wild type |
| PN2 | LPL | c.1160_1161insT (p.Lys387fs) | N2 | Wild type |
| PN3 | APOC2 | c.189C > A (p.Tyr63Ter) | N3 | Wild type |
| PN4 | LMF1 | c.895C > T (p.Gln299Ter) | N4 | Wild type |
| PN5 | GPIHBP1 | c.422G > A (p.Trp141Ter) | N5 | Wild type |
| PN6 | GPIHBP1 | c.417_433del (p.Pro140fs) | N6 | Wild type |
| PN7 | APOA5 | c.289C > T (p.Gln97Ter) | N7 | Wild type |
| PN8 | APOA5 | c.990_993del (p.Asp332fs) | N8 | Wild type |
| PN9 | GPD1 | c.219 + 1_219 + 3del | N9 | Wild type |

**Table 6. Parameter settings of the covaris ultrasonic fragmenter**

| Peak incident power (W) | Duty factor (%) | Cycles per burst (#) | Treatment time (s) | Temperature (°C) | Sample volume (µL) |
|---|---|---|---|---|---|
| 450 | 30 | 200 | 180 | 7 | 50 |

### 2.2 Library construction

### 2.2.1 End repair and A-tailing

1) transferred the fragmented sample to a 0.2 mL sterilized centrifuge tube, transferred the centrifuge tube onto ice, and then added the following reagents according to the table below.

**Table 7**

| Component | Single reaction system (µL) | Preparation of 11 Mixes (µL) |
|---|---|---|
| Fragmented sample | 50 | 550 |
| End Prep Mix 4 | 15 | 165 |
| Total volume | 65 | 715 |

2) After microcentrifugation, collected the liquid to the bottom of the tube for the following reaction.

**Table 8**

| Temperature | Time |
|---|---|
| Heated lid 105°C | On |
| 20°C | 15 min |
| 65°C | 15 min |
| 4°C | ∞ |

### 2.2.2 Adapter ligation

1) Thawed the ligase buffer, inverted to mix well, and placed on ice for later use.
2) Prepared the reaction system according to the table below, and on ice, dispensed the reagents into the above reaction tubes.

**Table 9**

| Component | Single reaction system (µL) | Preparation of 11 Mixes (µL) |
|---|---|---|
| Rapid Ligation buffer 2 | 25 | 275 |
| Rapid DNA ligase | 5 | 55 |
| Ultrapure water | 3 | 33 |
| Total volume | 33 | 363 |

It is recommended that the IDT UDI Adapter Kit be used as the adapter. When the cDNA input amount was ≤ 10 ng, the adapter was diluted 20-fold (diluted with Nuclease-Free Water) before use.
3) After carefully confirming the sample number, added 2 µL of Adapter to each sample, gently pipetted to mix well, and after microcentrifugation, collected the liquid to the bottom of the tube. Allowed to react according to the following amplification program.

**Table 10**

| Reaction temperature | Time |
|---|---|
| Heated lid 105°C | On |
| 20°C | 15 min |
| 4°C | ∞ |

### 2.2.3 Purification of post-ligation products

1) Equilibrated the magnetic beads at room temperature for 30 min and vortexed thoroughly to mix well, and took 80 µL into a new 1.5 mL centrifuge tube.
2) transferred 100 µL of the ligation product to the 1.5 mL centrifuge tube in step 2.2.2, vortexed to mix well, and incubated at room temperature for 5 min.
3) Placed the 1.5 mL centrifuge tube on a magnetic rack, let stand until the solution was completely clear, and discarded the supernatant.
4) Added 200 µL of freshly prepared 80% (v/v) ethanol, incubated at room temperature for 30 s, and aspirated and discarded the supernatant.
5) Repeated the previous step.
6) Microcentrifuged the 1.5 mL centrifuge tube and placed it on the magnetic rack, let stand for 1 min, discarded the residual solution, and air-dried with the lid open at room temperature until the ethanol had completely evaporated.
7) Added 22 µL of Nuclease-Free Water, vortexed to mix well, incubated at room temperature for 2 min, microcentrifuged and placed on the magnetic rack, and after the solution was completely clear, transferred 20 µL of the supernatant to a newly labeled 0.2 mL centrifuge tube.

### 2.2.4 Library amplification

1) Thawed the library construction amplification primers and the library construction amplification buffer, and inverted to mix well.
2) Prepared the reaction system according to the table below, and dispensed it into the above ligation purification product tubes.

**Table 11**

| Component | Single reaction system (µL) | Preparation of 11 Mixes (µL) |
|---|---|---|
| Purification products | 20 | 220 |
| PCR Primer Mix 3 for Illumina | 5 | 55 |
| VAHTS HiFi Amplification Mix | 25 | 275 |
| Total volume | 50 | 550 |

3) Gently pipetted to mix well, and after microcentrifugation, collected the liquid to the bottom of the tube. Amplification was carried out according to the following program:

**Table 12**

| **Temperature** | **Time** | **Number of cycles** |
|---|---|---|
| Heated lid 105°C | On | NA |
| 98°C | 3 min | 1 |
| 98°C | 20 s | 6 |
| 60°C | 15 s | |
| 72°C | 30 s | |
| 72°C | 5 min | 1 |
| 4°C | ∞ | 1 |

### 2.2.5 PCR product purification

1) Equilibrated the magnetic beads at room temperature for 30 min and vortexed thoroughly to mix well, and took 50 µL into a new 1.5 mL centrifuge tube.
2) transferred 50 µL of the PCR product to the 1.5 mL centrifuge tube in step 2.2.4, vortexed to mix well, and incubated at room temperature for 5 min.
3) Placed the 1.5 mL centrifuge tube on a magnetic rack, let stand until the solution was completely clear, and discarded the supernatant.
4) Added 200 µL of freshly prepared 80% ethanol, incubated at room temperature for 30 s, and discarded the supernatant.
5) Repeated the previous step.
6) Microcentrifuged the 1.5 mL centrifuge tube of step 5) and placed it on the magnetic rack, let stand for 1 min and then discarded the residual solution, and air-dried with the lid open at room temperature until the ethanol had completely volatilized.
7) Added 32 µL of Nuclease-Free Water, vortexed to mix well, incubated at room temperature for 2 min, microcentrifuged and place on the magnetic rack, and after the solution was completely clear, transferred 30 µL of the supernatant to a newly labeled 1.5 mL centrifuge tube, which was the library product.

### 2.2.6 Library quality control

1) Used the nucleic acid quantification kit Qubit^{™} dsDNA HS Assay Kit and its supporting instrument to determine the concentration of the DNA library, and the total amount of the DNA library should be ≥ 500 ng.
2) Used the DNA High Sensitivity Reagent Kit and its supporting instrument to determine the fragment length of the DNA library. The main peak should be within the range of 200-800 bp, and there should be no obvious small-fragment or large-fragment miscellaneous peaks

### 2.3 Target region enrichment

### 2.3.1 Library mixing and drying down

1) According to the records in Table 3 or Table 4, after carefully confirming the sample numbers, pooled the DNA libraries into a single tube in a new 1.5 mL LoBind centrifuge tube.
2) In a new 1.5 mL LoBind centrifuge tube, prepared the Blocker Master Mix according to the table below, and when there were multiple samples, prepared an extra 10%.

**Table 13**

| Component | Vol (µL) | Preparation of 11 Mixes (µL) |
|---|---|---|
| Human Cot DNA | 5 | 55 |
| xGen Universal Blocking Oligo - TS Mix | 1 | 11 |
| Total | 6 | 66 |

3) After mixing well, added 6 µL of Blocker Master Mix to each pool.
4) After fully vortexing to mix well, centrifuged to collect the Mix to the bottom of the tube, sealed with sealing film, punctured 5 small holes in the film, and placed in a vacuum concentrator to dry down.

### 2.3.2 Library redissolution, denaturation, and hybridization

1) In a new 1.5 mL LoBind centrifuge tube, prepared the Hybridization Master Mix according to the table below, and when there were multiple samples, prepared an additional amount.

**Table 14**

| Component | Volume (µL) | Preparation of 11 Mixes (µL) |
|---|---|---|
| xGen 2× Hybridization Buffer | 8.5 | 93.5 |
| xGen Hybridization Buffer Enhancer | 2.7 | 29.7 |
| Ultrapure water | 3.8 | 41.8 |
| Probe | 2 | 22 |
| Total volume | 17 | 187 |

2) After vortexing to mix well and centrifuging, added 17 µL of Hybridization Master Mix to the dried centrifuge tube (step 2.3.1), fully vortexed to mix well and then microcentrifuged to collect the solution at the bottom of the tube, and let to stand at room temperature for 5-10 min for redissolution. Then transferred all 17 µL of Mix to the pre-prepared 0.2 µL centrifuge tube and placed it in the PCR instrument, and after brief centrifugation, placed it on the PCR instrument to start the HYB program, incubated at 65°C overnight and performed the capture. The reaction system is as follows:

**Table 15**

| HYB program (heated lid set to 100°C) | |
|---|---|
| 95°C | 30 s |
| 65°C | Overnight |
| 65°C | ∞ |

### 2.3.3 Preparation of wash buffers

1) Prepared the 1× working solutions according to the table below, and no extra was needed.

**Table 16**

| Component | Buffer (µL) | UPDW (µL) |
|---|---|---|
| xGen 2× Bead Wash Buffer | 160 | 160 |
| xGen 10× Wash Buffer 1 | 16 | 144 |
| xGen 10× Wash Buffer 1 (metal bath) | 11 | 99 |
| xGen 10× Wash Buffer 2 | 16 | 144 |
| xGen 10× Wash Buffer 3 | 16 | 144 |
| xGen 10× Stringent Wash Buffer (metal bath) | 32 | 288 |

2) In a new 1.5 mL LoBind centrifuge tube, prepared the Bead Resuspension Mix according to the table below.

**Table 17**

| Component | Vol (µL) |
|---|---|
| xGen 2× Hybridization Buffer | 8.5 |
| xGen Hybridization Buffer Enhancer | 2.7 |
| Ultrapure water | 5.8 |
| Total | 17 |

### 2.3.4 Washing streptavidin M270 magnetic beads

1) Took out the magnetic beads at least 30 min in advance and equilibrated to room temperature, and then vortexed thoroughly to mix well for about 15 s.
2) According to the number of samples, respectively took 50 µL/pool into a new 1.5 mL centrifuge tube.
3) For each centrifuge tube, added 100 µL/pool of well-mixed 1× Bead Wash Buffer according to the number of CAPs, gently pipetted 10 times to mix well and then let stand on the magnetic rack, and after the solution was completely clear, aspirated and discarded the supernatant (allowed the magnetic beads to separate on the magnetic rack for 1 min).
4) Repeated the previous step 2 times.
5) Added 17 µL/pool of Bead Resuspension Mix to resuspend the magnetic beads, mixed thoroughly. The resuspension solution may be collected to the bottom of the well by microcentrifugation.
6) Aliquoted the above magnetic bead Mix, and put 17 µL per tube into new 0.2 µL centrifuge tubes.

### 2.3.5 Magnetic bead hybridization

1) At least 15 min in advance, placed 1× Wash Buffer 1 and 1× Stringent Wash Buffer on a 65°C metal bath for heating.
2) After the hybridization reaction was carried out overnight, took the sample out of the PCR instrument, and microcentrifuged the sample.
3) Turned off the HYB program, and turned on the WASH program.
4) transferred all 17 µL of magnetic beads (preheated) into the centrifuge tube for the hybridization reaction, vortexed to mix well, lightly centrifuged, and then placed into the PCR instrument.
5) Incubated for 45 min, and during the incubation process, vortexed to mix well every 12 min, avoiding splashing of the solution as much as possible.

**Table 18**

| WASH program (heated lid set to 70°C) | |
|---|---|
| 65°C | ∞ |

### 2.3.6 Heated washing

1) After the 45 min incubation was completed, took the sample out of the PCR instrument.
2) transferred 100 µL of preheated 1× Wash Buffer 1 into the sample, pipetted 10 times to mix well, and avoided generating too many bubbles.
3) transferred all of the above Mix to a new 1.5 mL centrifuge tube, let the sample stand on the magnetic rack for about 1 min, and after the solution was completely clear, aspirated and discarded the supernatant.
4) Removed the sample from the magnetic rack, transferred 150 µL of preheated 1× Stringent Wash Buffer into the sample, and pipetted 10 times to mix well.
5) After placing on the metal bath and incubating at 65°C for 5 min, let the sample stand on the magnetic rack, and after the solution was completely clear, aspirated and discarded the supernatant;
6) Repeated steps 4) to 5).

### 2.3.7 Room-temperature washing

1) Added 150 µL of well-mixed 1× Wash Buffer 1, vortexed to mix well and then incubated for 2 min, during which let stand for 30 s and shook for 30 s, and after brief centrifugation, let stand on the magnetic rack (about 1 min), and after the solution was completely clear, aspirated and discarded the supernatant.
2) Added 150 µL of well-mixed 1× Wash Buffer 2, vortexed to mix well and then incubated for 2 min, during which let stand for 30 s and shook for 30 s, and after brief centrifugation, let stand on the magnetic rack (about 1 min), and after the solution was completely clear, aspirated and discarded the supernatant.
3) Added 150 µL of well-mixed 1× Wash Buffer 3, vortexed to mix well and then incubated for 2 min, during which let stand for 30 s and shook for 30 s, and after brief centrifugation, let stand on the magnetic rack (about 1 min), and after the solution was completely clear, aspirated and discarded the supernatant.
4) After centrifuging again, used a 10 µL pipette tip to aspirate and discarded all residual 1× Wash Buffer 3 liquid.
5) Removed the sample from the magnetic rack, added 20 µL of ultrapure water into each centrifuge tube, pipetted 10 times to mix well and resuspended the magnetic beads, then carefully confirmed the sample number, and transferred all resuspension liquid to a newly labeled 0.2 mL centrifuge tube.

### 2.3.8 PCR amplification

1) In a new LoBind centrifuge tube, prepared the Amplification Reaction Mix, and when there were multiple samples prepared an extra 20%.

**Table 19**

| Component | Vol (µL) |
|---|---|
| KAPA HiFi HotStart ReadyMix | 25 |
| P7 Primer (SEQ ID NO.426: AATGATACGGCGACCACCGA) (10 µM) | 2.5 |
| P5 Primer (SEQ ID NO.427: CAAGCAGAAGACGGCATACGA) (10 µM) | 2.5 |
| Total | 30 |

2) Transferred 30 µL of Amplification Reaction Mix to the 0.2 mL centrifuge tube (step 2.3.8), vortexed to mix well, microcentrifuged, and then placed into the PCR instrument and allowed to react according to the following program.

**Table 20**

| Reaction temperature | Reaction time | Number of cycles |
|---|---|---|
| 98°C | 45 s | 1 |
| 98°C | 15 s | 10 |
| 60°C | 30 s | |
| 72°C | 30 s | |
| 72°C | 1 min | 1 |
| 4°C | ∞ | 1 |

### 2.3.9 Post-PCR purification

1) Prepared 400 µL of fresh 80% ethanol for each sample, and when there were multiple samples, prepared an extra 10%.
2) Dispensed the AMpure XP Beads already equilibrated to room temperature into new 1.5 mL centrifuge tubes, 75 µL each.
3) After carefully checking the numbers, transferred all of the amplification product to the 1.5 mL centrifuge tube containing magnetic beads, vortexed to mix well and microcentrifuged, incubated at room temperature for 5-10 min and then let stand on the magnetic rack, and after the solution was completely clear, aspirated and discarded the supernatant.
4) Added 200 µL of freshly prepared 80% ethanol, let stand for 1 min, and then aspirated and discarded the supernatant.
5) Repeated step 4).
6) Kept the sample on the magnetic rack, and air-dried the magnetic beads for 1-3 min.
7) Added 27 µL of ultrapure water to the centrifuge tube, vortexed thoroughly to mix well and microcentrifuged, incubated at room temperature for 5 min, and placed the centrifuge tube on the magnetic rack.
8) After carefully confirming the sample number, transferred 25 µL of the supernatant to a labeled new 1.5 mL centrifuge tube.

### 2.4 Sequencing

Sequencing of the above 10 samples was performed according to the manufacturer's instructions for the NovaSeq 6000 (Illumina) sequencing system and NovaSeq 6000 v2 kit.

The sequencing data were analyzed by bioinformatics methods, and the detection results are as shown in attached Table 21 and Table 22:

**Table 21. Detection results for 10 mutant gDNA samples clearly containing known variant types of the 6 genes**

| Sample number | Gene | Variant information | Detection result (negative/positive) | Mutation frequency (%) |
|---|---|---|---|---|
| PN1 | LPL | c.249 + 1G > A | Positive | 61.52 |
| PN2 | LPL | c.1160_1161insT (p.Lys387fs) | Positive | 50.84 |
| PN3 | APOC2 | c.189C > A (p.Tyr63Ter) | Positive | 33.04 |
| PN4 | LMF1 | c.895C > T (p.Gln299Ter) | Positive | 45.99 |
| PN5 | GPIHBP1 | c.422G > A (p.Trp141Ter) | Positive | 28.36 |
| PN6 | GPIHBP1 | c.417_433del (p.Pro140fs) | Positive | 42.82 |
| PN7 | APOA5 | c.289C > T (p.Gln97Ter) | Positive | 59.61 |
| PN8 | APOA5 | c.990_993del (p.Asp332fs) | Positive | 56.64 |
| PN9 | GPD1 | c.219 + 1_219 + 3del | Positive | 48.15 |
| PN10 | GPD1 | c.751dup (p.Thr251fs) | Positive | 47.30 |

**Table 22. Detection results for 10 wild-type gDNA samples not containing any variant type of the 6 genes**

| Sample number | Detection result (negative/positive) |
|---|---|
| N1 | Negative |
| N2 | Negative |
| N3 | Negative |
| N4 | Negative |
| N5 | Negative |
| N6 | Negative |
| N7 | Negative |
| N8 | Negative |
| N9 | Negative |
| N10 | Negative |

From the above data, it can be seen that the method of this example showed 100% sensitivity and 100% specificity for detecting variants in the 6 characteristic FCS genes (LPL, APOC2, LMF1, GPIHBP1, APOA5, and GPD1). It has extremely high detection accuracy.

Overall, the embodiments of the present application, through a capture sequencing method, designed target capture probes covering all exons and hotspot intronic regions of 6 FCS-related genes (*LPL*, *APOC2*, *LMF1*, *GPIHBP1*, *APOA5*, and *GPD1*), and through detection of the variant types of the above 6 genes closely related to FCS, achieved precise detection of FCS, which is of great significance to the clinical diagnosis and treatment of FCS.

The technical features of the above-described embodiments and examples may be combined in any suitable manner, and for simplicity of description, not all possible combinations of the technical features in the above embodiments and examples have been described; however, as long as the combinations of these technical features are not contradictory, they shall all be considered to fall within the scope described in this specification.

The above-described examples merely illustrate several embodiments of the present invention, to facilitate concrete and detailed understanding of the technical solution of the present invention, but this shall not therefore be understood as a limitation on the scope of patent protection of the invention. It should be pointed out that, for a person of ordinary skill in the art, without departing from the concept of the present invention, several modifications and improvements may also be made, and these all belong to the protection scope of the present invention. In addition, it should be understood that, after reading the above disclosure of the present invention, a person skilled in the art may make various changes or modifications to the present invention, and any equivalent forms obtained in a similar way fall within the protection scope of the present application. It should also be understood that technical solutions obtained by a person skilled in the art based on the technical solution provided by the present invention, through logical analysis, reasoning, or limited experiments, are all within the protection scope of the claims appended to the present invention. Therefore, the protection scope of the patent of the present invention shall be subject to the content of the appended claims, and the specification may be used to interpret the content of the claims.

## Claims

1. A capture probe, which comprises one or more probe sets from a probe set 1 to a probe set 6;
probe set 1 comprises one or more probes selected from probes 1 to 26;
probe set 2 comprises one or more probes selected from probes 27 to 64;
probe set 3 comprises one or more probes selected from probes 65 to 112;
probe set 4 comprises one or more probes selected from probes 113 to 139;
probe set 5 comprises one or more probes selected from probes 140 to 382;
probe set 6 comprises one or more probes selected from probes 383 to 425;
the probes 1 to 425 are constructed for target gene fragments located between different start positions and end positions on the following chromosomes and are complementary to and paired with said target gene fragments:
| Probe number | Chromosome | Start position | End position |
|---|---|---|---|
| Probe 1 | chr11 | 116660063 | 116660183; |
| Probe 2 | chr11 | 116660168 | 116660288; |
| Probe 3 | chr11 | 116660273 | 116660393; |
| Probe 4 | chr11 | 116660378 | 116660498; |
| Probe 5 | chr11 | 116660483 | 116660603; |
| Probe 6 | chr11 | 116660588 | 116660708; |
| Probe 7 | chr11 | 116660693 | 116660813; |
| Probe 8 | chr11 | 116660798 | 116660918; |
| Probe 9 | chr11 | 116660903 | 116661023; |
| Probe 10 | chr11 | 116661008 | 116661128; |
| Probe 11 | chr11 | 116661113 | 116661233; |
| Probe 12 | chr11 | 116661218 | 116661338; |
| Probe 13 | chr11 | 116661323 | 116661443; |
| Probe 14 | chr11 | 116661428 | 116661548; |
| Probe 15 | chr11 | 116661533 | 116661653; |
| Probe 16 | chr11 | 116661638 | 116661758; |
| Probe 17 | chr11 | 116661743 | 116661863; |
| Probe 18 | chr11 | 116662282 | 116662402; |
| Probe 19 | chr11 | 116662387 | 116662507; |
| Probe 20 | chr11 | 116662508 | 116662628; |
| Probe 21 | chr11 | 116663076 | 116663196; |
| Probe 22 | chr11 | 116663181 | 116663301; |
| Probe 23 | chr11 | 116663286 | 116663406; |
| Probe 24 | chr11 | 116663391 | 116663511; |
| Probe 25 | chr11 | 116663496 | 116663616; |
| Probe 26 | chr11 | 116663601 | 116663721; |
| Probe 27 | chr12 | 50497271 | 50497391; |
| Probe 28 | chr12 | 50497376 | 50497496; |
| Probe 29 | chr12 | 50497481 | 50497601; |
| Probe 30 | chr12 | 50497586 | 50497706; |
| Probe 31 | chr12 | 50497691 | 50497811; |
| Probe 32 | chr12 | 50497796 | 50497916; |
| Probe 33 | chr12 | 50498337 | 50498457; |
| Probe 34 | chr12 | 50498442 | 50498562; |
| Probe 35 | chr12 | 50499311 | 50499431; |
| Probe 36 | chr12 | 50499416 | 50499536; |
| Probe 37 | chr12 | 50500051 | 50500171; |
| Probe 38 | chr12 | 50500156 | 50500276; |
| Probe 39 | chr12 | 50500568 | 50500688; |
| Probe 40 | chr12 | 50500673 | 50500793; |
| Probe 41 | chr12 | 50501330 | 50501450; |
| Probe 42 | chr12 | 50501435 | 50501555; |
| Probe 43 | chr12 | 50501540 | 50501660; |
| Probe 44 | chr12 | 50501787 | 50501907; |
| Probe 45 | chr12 | 50501892 | 50502012; |
| Probe 46 | chr12 | 50503186 | 50503306; |
| Probe 47 | chr12 | 50503291 | 50503411; |
| Probe 48 | chr12 | 50503396 | 50503516; |
| Probe 49 | chr12 | 50503501 | 50503621; |
| Probe 50 | chr12 | 50503606 | 50503726; |
| Probe 51 | chr12 | 50503711 | 50503831; |
| Probe 52 | chr12 | 50503816 | 50503936; |
| Probe 53 | chr12 | 50503921 | 50504041; |
| Probe 54 | chr12 | 50504026 | 50504146; |
| Probe 55 | chr12 | 50504131 | 50504251; |
| Probe 56 | chr12 | 50504236 | 50504356; |
| Probe 57 | chr12 | 50504341 | 50504461; |
| Probe 58 | chr12 | 50504446 | 50504566; |
| Probe 59 | chr12 | 50504551 | 50504671; |
| Probe 60 | chr12 | 50504656 | 50504776; |
| Probe 61 | chr12 | 50504761 | 50504881; |
| Probe 62 | chr12 | 50504866 | 50504986; |
| Probe 63 | chr12 | 50504971 | 50505091; |
| Probe 64 | chr12 | 50505076 | 50505196; |
| Probe 65 | chr16 | 903614 | 903734; |
| Probe 66 | chr16 | 903719 | 903839; |
| Probe 67 | chr16 | 903824 | 903944; |
| Probe 68 | chr16 | 903929 | 904049; |
| Probe 69 | chr16 | 904034 | 904154; |
| Probe 70 | chr16 | 904139 | 904259; |
| Probe 71 | chr16 | 904244 | 904364; |
| Probe 72 | chr16 | 904349 | 904469; |
| Probe 73 | chr16 | 904454 | 904574; |
| Probe 74 | chr16 | 904559 | 904679; |
| Probe 75 | chr16 | 904664 | 904784; |
| Probe 76 | chr16 | 905087 | 905207; |
| Probe 77 | chr16 | 905192 | 905312; |
| Probe 78 | chr16 | 918924 | 919044; |
| Probe 79 | chr16 | 919029 | 919149; |
| Probe 80 | chr16 | 919863 | 919983; |
| Probe 81 | chr16 | 919968 | 920088; |
| Probe 82 | chr16 | 920709 | 920829; |
| Probe 83 | chr16 | 920814 | 920934; |
| Probe 84 | chr16 | 921141 | 921261; |
| Probe 85 | chr16 | 921246 | 921366; |
| Probe 86 | chr16 | 929550 | 929670; |
| Probe 87 | chr16 | 929655 | 929775; |
| Probe 88 | chr16 | 942987 | 943107; |
| Probe 89 | chr16 | 960911 | 961031; |
| Probe 90 | chr16 | 961016 | 961136; |
| Probe 91 | chr16 | 981622 | 981742; |
| Probe 92 | chr16 | 981727 | 981847; |
| Probe 93 | chr16 | 983971 | 984091; |
| Probe 94 | chr16 | 984076 | 984196; |
| Probe 95 | chr16 | 984170 | 984290; |
| Probe 96 | chr16 | 1004337 | 1004457; |
| Probe 97 | chr16 | 1004442 | 1004562; |
| Probe 98 | chr16 | 1004547 | 1004667; |
| Probe 99 | chr16 | 1004652 | 1004772; |
| Probe 100 | chr16 | 1020768 | 1020888; |
| Probe 101 | chr16 | 1020873 | 1020993; |
| Probe 102 | chr16 | 1020978 | 1021098; |
| Probe 103 | chr16 | 1031125 | 1031245; |
| Probe 104 | chr16 | 1031230 | 1031350; |
| Probe 105 | chr16 | 1031335 | 1031455; |
| Probe 106 | chr16 | 1031440 | 1031560; |
| Probe 107 | chr16 | 1031545 | 1031665; |
| Probe 108 | chr16 | 1031650 | 1031770; |
| Probe 109 | chr16 | 1031755 | 1031875; |
| Probe 110 | chr16 | 1031860 | 1031980; |
| Probe 111 | chr16 | 1031965 | 1032085; |
| Probe 112 | chr16 | 1032070 | 1032190; |
| Probe 113 | chr19 | 45448788 | 45448908; |
| Probe 114 | chr19 | 45448893 | 45449013; |
| Probe 115 | chr19 | 45448998 | 45449118; |
| Probe 116 | chr19 | 45449103 | 45449223; |
| Probe 117 | chr19 | 45449208 | 45449328; |
| Probe 118 | chr19 | 45449313 | 45449433; |
| Probe 119 | chr19 | 45449418 | 45449538; |
| Probe 120 | chr19 | 45449628 | 45449748; |
| Probe 121 | chr19 | 45449733 | 45449853; |
| Probe 122 | chr19 | 45450048 | 45450168; |
| Probe 123 | chr19 | 45450363 | 45450483; |
| Probe 124 | chr19 | 45450468 | 45450588; |
| Probe 125 | chr19 | 45450573 | 45450693; |
| Probe 126 | chr19 | 45450678 | 45450798; |
| Probe 127 | chr19 | 45451518 | 45451638; |
| Probe 128 | chr19 | 45451623 | 45451743; |
| Probe 129 | chr19 | 45451728 | 45451848; |
| Probe 130 | chr19 | 45451833 | 45451953; |
| Probe 131 | chr19 | 45451938 | 45452058; |
| Probe 132 | chr19 | 45452043 | 45452163; |
| Probe 133 | chr19 | 45452148 | 45452268; |
| Probe 134 | chr19 | 45452253 | 45452373; |
| Probe 135 | chr19 | 45452358 | 45452478; |
| Probe 136 | chr19 | 45452463 | 45452583; |
| Probe 137 | chr19 | 45452568 | 45452688; |
| Probe 138 | chr19 | 45452673 | 45452793; |
| Probe 139 | chr19 | 45452778 | 45452898; |
| Probe 140 | chr8 | 19796244 | 19796364; |
| Probe 141 | chr8 | 19796349 | 19796469; |
| Probe 142 | chr8 | 19796454 | 19796574; |
| Probe 143 | chr8 | 19796559 | 19796679; |
| Probe 144 | chr8 | 19796664 | 19796784; |
| Probe 145 | chr8 | 19796769 | 19796889; |
| Probe 146 | chr8 | 19796874 | 19796994; |
| Probe 147 | chr8 | 19796979 | 19797099; |
| Probe 148 | chr8 | 19797084 | 19797204; |
| Probe 149 | chr8 | 19797189 | 19797309; |
| Probe 150 | chr8 | 19797294 | 19797414; |
| Probe 151 | chr8 | 19797399 | 19797519; |
| Probe 152 | chr8 | 19797504 | 19797624; |
| Probe 153 | chr8 | 19797609 | 19797729; |
| Probe 154 | chr8 | 19797714 | 19797834; |
| Probe 155 | chr8 | 19797819 | 19797939; |
| Probe 156 | chr8 | 19797924 | 19798044; |
| Probe 157 | chr8 | 19798029 | 19798149; |
| Probe 158 | chr8 | 19798134 | 19798254; |
| Probe 159 | chr8 | 19798239 | 19798359; |
| Probe 160 | chr8 | 19798344 | 19798464; |
| Probe 161 | chr8 | 19798449 | 19798569; |
| Probe 162 | chr8 | 19798554 | 19798674; |
| Probe 163 | chr8 | 19798659 | 19798779; |
| Probe 164 | chr8 | 19798764 | 19798884; |
| Probe 165 | chr8 | 19798869 | 19798989; |
| Probe 166 | chr8 | 19798974 | 19799094; |
| Probe 167 | chr8 | 19799079 | 19799199; |
| Probe 168 | chr8 | 19799184 | 19799304; |
| Probe 169 | chr8 | 19799289 | 19799409; |
| Probe 170 | chr8 | 19799394 | 19799514; |
| Probe 171 | chr8 | 19799499 | 19799619; |
| Probe 172 | chr8 | 19799604 | 19799724; |
| Probe 173 | chr8 | 19799709 | 19799829; |
| Probe 174 | chr8 | 19799814 | 19799934; |
| Probe 175 | chr8 | 19799919 | 19800039; |
| Probe 176 | chr8 | 19800024 | 19800144; |
| Probe 177 | chr8 | 19800129 | 19800249; |
| Probe 178 | chr8 | 19800234 | 19800354; |
| Probe 179 | chr8 | 19800339 | 19800459; |
| Probe 180 | chr8 | 19800444 | 19800564; |
| Probe 181 | chr8 | 19800549 | 19800669; |
| Probe 182 | chr8 | 19800654 | 19800774; |
| Probe 183 | chr8 | 19800759 | 19800879; |
| Probe 184 | chr8 | 19800864 | 19800984; |
| Probe 185 | chr8 | 19800969 | 19801089; |
| Probe 186 | chr8 | 19801074 | 19801194; |
| Probe 187 | chr8 | 19801179 | 19801299; |
| Probe 188 | chr8 | 19801284 | 19801404; |
| Probe 189 | chr8 | 19801704 | 19801824; |
| Probe 190 | chr8 | 19801809 | 19801929; |
| Probe 191 | chr8 | 19801914 | 19802034; |
| Probe 192 | chr8 | 19802019 | 19802139; |
| Probe 193 | chr8 | 19802124 | 19802244; |
| Probe 194 | chr8 | 19802229 | 19802349; |
| Probe 195 | chr8 | 19802334 | 19802454; |
| Probe 196 | chr8 | 19802439 | 19802559; |
| Probe 197 | chr8 | 19802544 | 19802664; |
| Probe 198 | chr8 | 19802649 | 19802769; |
| Probe 199 | chr8 | 19802754 | 19802874; |
| Probe 200 | chr8 | 19802859 | 19802979; |
| Probe 201 | chr8 | 19802964 | 19803084; |
| Probe 202 | chr8 | 19803069 | 19803189; |
| Probe 203 | chr8 | 19803174 | 19803294; |
| Probe 204 | chr8 | 19803279 | 19803399; |
| Probe 205 | chr8 | 19803384 | 19803504; |
| Probe 206 | chr8 | 19803489 | 19803609; |
| Probe 207 | chr8 | 19803594 | 19803714; |
| Probe 208 | chr8 | 19803699 | 19803819; |
| Probe 209 | chr8 | 19803804 | 19803924; |
| Probe 210 | chr8 | 19803909 | 19804029; |
| Probe 211 | chr8 | 19804014 | 19804134; |
| Probe 212 | chr8 | 19804119 | 19804239; |
| Probe 213 | chr8 | 19804224 | 19804344; |
| Probe 214 | chr8 | 19804329 | 19804449; |
| Probe 215 | chr8 | 19804434 | 19804554; |
| Probe 216 | chr8 | 19804539 | 19804659; |
| Probe 217 | chr8 | 19804644 | 19804764; |
| Probe 218 | chr8 | 19805064 | 19805184; |
| Probe 219 | chr8 | 19805169 | 19805289; |
| Probe 220 | chr8 | 19805274 | 19805394; |
| Probe 221 | chr8 | 19805379 | 19805499; |
| Probe 222 | chr8 | 19805484 | 19805604; |
| Probe 223 | chr8 | 19805589 | 19805709; |
| Probe 224 | chr8 | 19805694 | 19805814; |
| Probe 225 | chr8 | 19805799 | 19805919; |
| Probe 226 | chr8 | 19805904 | 19806024; |
| Probe 227 | chr8 | 19806009 | 19806129; |
| Probe 228 | chr8 | 19806114 | 19806234; |
| Probe 229 | chr8 | 19806219 | 19806339; |
| Probe 230 | chr8 | 19806324 | 19806444; |
| Probe 231 | chr8 | 19806429 | 19806549; |
| Probe 232 | chr8 | 19806534 | 19806654; |
| Probe 233 | chr8 | 19806639 | 19806759; |
| Probe 234 | chr8 | 19806744 | 19806864; |
| Probe 235 | chr8 | 19806849 | 19806969; |
| Probe 236 | chr8 | 19807269 | 19807389; |
| Probe 237 | chr8 | 19807374 | 19807494; |
| Probe 238 | chr8 | 19807479 | 19807599; |
| Probe 239 | chr8 | 19807584 | 19807704; |
| Probe 240 | chr8 | 19807689 | 19807809; |
| Probe 241 | chr8 | 19807794 | 19807914; |
| Probe 242 | chr8 | 19807899 | 19808019; |
| Probe 243 | chr8 | 19808319 | 19808439; |
| Probe 244 | chr8 | 19808424 | 19808544; |
| Probe 245 | chr8 | 19808529 | 19808649; |
| Probe 246 | chr8 | 19808634 | 19808754; |
| Probe 247 | chr8 | 19808739 | 19808859; |
| Probe 248 | chr8 | 19808844 | 19808964; |
| Probe 249 | chr8 | 19808949 | 19809069; |
| Probe 250 | chr8 | 19809054 | 19809174; |
| Probe 251 | chr8 | 19809159 | 19809279; |
| Probe 252 | chr8 | 19809264 | 19809384; |
| Probe 253 | chr8 | 19809369 | 19809489; |
| Probe 254 | chr8 | 19809474 | 19809594; |
| Probe 255 | chr8 | 19809579 | 19809699; |
| Probe 256 | chr8 | 19809684 | 19809804; |
| Probe 257 | chr8 | 19809789 | 19809909; |
| Probe 258 | chr8 | 19809894 | 19810014; |
| Probe 259 | chr8 | 19809999 | 19810119; |
| Probe 260 | chr8 | 19810104 | 19810224; |
| Probe 261 | chr8 | 19810209 | 19810329; |
| Probe 262 | chr8 | 19810314 | 19810434; |
| Probe 263 | chr8 | 19810419 | 19810539; |
| Probe 264 | chr8 | 19810524 | 19810644; |
| Probe 265 | chr8 | 19810629 | 19810749; |
| Probe 266 | chr8 | 19810734 | 19810854; |
| Probe 267 | chr8 | 19810839 | 19810959; |
| Probe 268 | chr8 | 19810944 | 19811064; |
| Probe 269 | chr8 | 19811049 | 19811169; |
| Probe 270 | chr8 | 19811154 | 19811274; |
| Probe 271 | chr8 | 19811259 | 19811379; |
| Probe 272 | chr8 | 19811364 | 19811484; |
| Probe 273 | chr8 | 19811469 | 19811589; |
| Probe 274 | chr8 | 19811574 | 19811694; |
| Probe 275 | chr8 | 19811679 | 19811799; |
| Probe 276 | chr8 | 19811784 | 19811904; |
| Probe 277 | chr8 | 19811889 | 19812009; |
| Probe 278 | chr8 | 19811994 | 19812114; |
| Probe 279 | chr8 | 19812099 | 19812219; |
| Probe 280 | chr8 | 19812204 | 19812324; |
| Probe 281 | chr8 | 19812309 | 19812429; |
| Probe 282 | chr8 | 19812519 | 19812639; |
| Probe 283 | chr8 | 19812624 | 19812744; |
| Probe 284 | chr8 | 19812729 | 19812849; |
| Probe 285 | chr8 | 19812834 | 19812954; |
| Probe 286 | chr8 | 19812939 | 19813059; |
| Probe 287 | chr8 | 19813044 | 19813164; |
| Probe 288 | chr8 | 19813149 | 19813269; |
| Probe 289 | chr8 | 19813254 | 19813374; |
| Probe 290 | chr8 | 19813359 | 19813479; |
| Probe 291 | chr8 | 19813464 | 19813584; |
| Probe 292 | chr8 | 19813569 | 19813689; |
| Probe 293 | chr8 | 19813674 | 19813794; |
| Probe 294 | chr8 | 19813779 | 19813899; |
| Probe 295 | chr8 | 19813884 | 19814004; |
| Probe 296 | chr8 | 19814094 | 19814214; |
| Probe 297 | chr8 | 19814199 | 19814319; |
| Probe 298 | chr8 | 19814304 | 19814424; |
| Probe 299 | chr8 | 19814619 | 19814739; |
| Probe 300 | chr8 | 19814724 | 19814844; |
| Probe 301 | chr8 | 19814829 | 19814949; |
| Probe 302 | chr8 | 19814934 | 19815054; |
| Probe 303 | chr8 | 19815039 | 19815159; |
| Probe 304 | chr8 | 19815144 | 19815264; |
| Probe 305 | chr8 | 19815249 | 19815369; |
| Probe 306 | chr8 | 19815354 | 19815474; |
| Probe 307 | chr8 | 19815459 | 19815579; |
| Probe 308 | chr8 | 19815774 | 19815894; |
| Probe 309 | chr8 | 19815879 | 19815999; |
| Probe 310 | chr8 | 19815984 | 19816104; |
| Probe 311 | chr8 | 19816194 | 19816314; |
| Probe 312 | chr8 | 19816299 | 19816419; |
| Probe 313 | chr8 | 19816404 | 19816524; |
| Probe 314 | chr8 | 19816509 | 19816629; |
| Probe 315 | chr8 | 19816614 | 19816734; |
| Probe 316 | chr8 | 19816719 | 19816839; |
| Probe 317 | chr8 | 19816824 | 19816944; |
| Probe 318 | chr8 | 19816929 | 19817049; |
| Probe 319 | chr8 | 19817034 | 19817154; |
| Probe 320 | chr8 | 19817139 | 19817259; |
| Probe 321 | chr8 | 19817559 | 19817679; |
| Probe 322 | chr8 | 19817664 | 19817784; |
| Probe 323 | chr8 | 19817769 | 19817889; |
| Probe 324 | chr8 | 19817874 | 19817994; |
| Probe 325 | chr8 | 19817979 | 19818099; |
| Probe 326 | chr8 | 19818084 | 19818204; |
| Probe 327 | chr8 | 19818189 | 19818309; |
| Probe 328 | chr8 | 19818294 | 19818414; |
| Probe 329 | chr8 | 19818399 | 19818519; |
| Probe 330 | chr8 | 19818504 | 19818624; |
| Probe 331 | chr8 | 19818609 | 19818729; |
| Probe 332 | chr8 | 19818714 | 19818834; |
| Probe 333 | chr8 | 19818819 | 19818939; |
| Probe 334 | chr8 | 19818924 | 19819044; |
| Probe 335 | chr8 | 19819029 | 19819149; |
| Probe 336 | chr8 | 19819134 | 19819254; |
| Probe 337 | chr8 | 19819239 | 19819359; |
| Probe 338 | chr8 | 19819344 | 19819464; |
| Probe 339 | chr8 | 19819449 | 19819569; |
| Probe 340 | chr8 | 19819554 | 19819674; |
| Probe 341 | chr8 | 19819659 | 19819779; |
| Probe 342 | chr8 | 19819764 | 19819884; |
| Probe 343 | chr8 | 19819869 | 19819989; |
| Probe 344 | chr8 | 19819974 | 19820094; |
| Probe 345 | chr8 | 19820079 | 19820199; |
| Probe 346 | chr8 | 19820184 | 19820304; |
| Probe 347 | chr8 | 19820289 | 19820409; |
| Probe 348 | chr8 | 19820394 | 19820514; |
| Probe 349 | chr8 | 19820499 | 19820619; |
| Probe 350 | chr8 | 19820604 | 19820724; |
| Probe 351 | chr8 | 19820919 | 19821039; |
| Probe 352 | chr8 | 19821024 | 19821144; |
| Probe 353 | chr8 | 19821129 | 19821249; |
| Probe 354 | chr8 | 19821234 | 19821354; |
| Probe 355 | chr8 | 19821339 | 19821459; |
| Probe 356 | chr8 | 19821759 | 19821879; |
| Probe 357 | chr8 | 19821864 | 19821984; |
| Probe 358 | chr8 | 19821969 | 19822089; |
| Probe 359 | chr8 | 19822284 | 19822404; |
| Probe 360 | chr8 | 19822389 | 19822509; |
| Probe 361 | chr8 | 19822494 | 19822614; |
| Probe 362 | chr8 | 19822599 | 19822719; |
| Probe 363 | chr8 | 19822704 | 19822824; |
| Probe 364 | chr8 | 19822809 | 19822929; |
| Probe 365 | chr8 | 19822914 | 19823034; |
| Probe 366 | chr8 | 19823019 | 19823139; |
| Probe 367 | chr8 | 19823124 | 19823244; |
| Probe 368 | chr8 | 19823229 | 19823349; |
| Probe 369 | chr8 | 19823334 | 19823454; |
| Probe 370 | chr8 | 19823439 | 19823559; |
| Probe 371 | chr8 | 19823544 | 19823664; |
| Probe 372 | chr8 | 19823649 | 19823769; |
| Probe 373 | chr8 | 19823754 | 19823874; |
| Probe 374 | chr8 | 19823859 | 19823979; |
| Probe 375 | chr8 | 19823964 | 19824084; |
| Probe 376 | chr8 | 19824069 | 19824189; |
| Probe 377 | chr8 | 19824174 | 19824294; |
| Probe 378 | chr8 | 19824279 | 19824399; |
| Probe 379 | chr8 | 19824384 | 19824504; |
| Probe 380 | chr8 | 19824489 | 19824609; |
| Probe 381 | chr8 | 19824594 | 19824714; |
| Probe 382 | chr8 | 19824699 | 19824819; |
| Probe 383 | chr8 | 144294573 | 144294693; |
| Probe 384 | chr8 | 144294678 | 144294798; |
| Probe 385 | chr8 | 144294783 | 144294903; |
| Probe 386 | chr8 | 144294888 | 144295008; |
| Probe 387 | chr8 | 144294993 | 144295113; |
| Probe 388 | chr8 | 144295098 | 144295218; |
| Probe 389 | chr8 | 144295203 | 144295323; |
| Probe 390 | chr8 | 144295308 | 144295428; |
| Probe 391 | chr8 | 144295413 | 144295533; |
| Probe 392 | chr8 | 144295518 | 144295638; |
| Probe 393 | chr8 | 144295623 | 144295743; |
| Probe 394 | chr8 | 144295728 | 144295848; |
| Probe 395 | chr8 | 144295833 | 144295953; |
| Probe 396 | chr8 | 144295938 | 144296058; |
| Probe 397 | chr8 | 144296043 | 144296163; |
| Probe 398 | chr8 | 144296148 | 144296268; |
| Probe 399 | chr8 | 144296253 | 144296373; |
| Probe 400 | chr8 | 144296358 | 144296478; |
| Probe 401 | chr8 | 144296463 | 144296583; |
| Probe 402 | chr8 | 144296568 | 144296688; |
| Probe 403 | chr8 | 144296673 | 144296793; |
| Probe 404 | chr8 | 144296778 | 144296898; |
| Probe 405 | chr8 | 144296883 | 144297003; |
| Probe 406 | chr8 | 144296988 | 144297108; |
| Probe 407 | chr8 | 144297093 | 144297213; |
| Probe 408 | chr8 | 144297198 | 144297318; |
| Probe 409 | chr8 | 144297303 | 144297423; |
| Probe 410 | chr8 | 144297408 | 144297528; |
| Probe 411 | chr8 | 144297513 | 144297633; |
| Probe 412 | chr8 | 144297618 | 144297738; |
| Probe 413 | chr8 | 144297723 | 144297843; |
| Probe 414 | chr8 | 144297828 | 144297948; |
| Probe 415 | chr8 | 144297933 | 144298053; |
| Probe 416 | chr8 | 144298038 | 144298158; |
| Probe 417 | chr8 | 144298143 | 144298263; |
| Probe 418 | chr8 | 144298248 | 144298368; |
| Probe 419 | chr8 | 144298353 | 144298473; |
| Probe 420 | chr8 | 144298458 | 144298578; |
| Probe 421 | chr8 | 144298563 | 144298683; |
| Probe 422 | chr8 | 144298668 | 144298788; |
| Probe 423 | chr8 | 144298773 | 144298893; |
| Probe 424 | chr8 | 144298878 | 144298998; |
| Probe 425 | chr8 | 144298983 | 144299103; |
the reference genome is Hg19.

2. The capture probe according to claim 1, which comprises a plurality of probe sets selected from a probe set 1 to a probe set 6;
the probe set 1 comprises a plurality of probes selected from the probes 1 to 26;
the probe set 2 comprises a plurality of probes selected from the probes 27 to 64;
the probe set 3 comprises a plurality of probes selected from the probes 65 to 112;
the probe set 4 comprises a plurality of probes selected from the probes 113 to 139;
the probe set 5 comprises a plurality of probes selected from the probes 140 to 382;
the probe set 6 comprises a plurality of probes selected from the probes 383 to 425.

3. The capture probe according to claim 2, which comprises the probe set 1 to the probe set 6;
the probe set 1 comprises the probes 1 to 26;
the probe set 2 comprises the probes 27 to 64;
the probe set 3 comprises the probes 65 to 112;
the probe set 4 comprises the probes 113 to 139;
the probe set 5 comprises the probes 140 to 382;
the probe set 6 comprises the probes 383 to 425.

4. Use of the capture probe according to any one of claims 1 to 3 in preparing a familial chylomicronemia syndrome detection kit.

5. A familial chylomicronemia syndrome detection kit, which comprises the capture probe according to any one of claims 1 to 3.

6. The familial chylomicronemia syndrome detection kit according to claim 5, which further comprises one or more of a nucleic acid extraction reagent, a DNA library construction reagent, a sample and library quantification reagent, a fragment quality control reagent, a hybridization capture reagent, a nucleic acid purification reagent, a target gene fragment amplification reagent, and a sequencing reagent.

7. Use of the capture probe according to any one of claims 1 to 3 in constructing a familial chylomicronemia syndrome-related gene mutation detection library.

8. Use of the detection kit according to claim 5 or 6 in constructing a familial chylomicronemia syndrome-related gene mutation detection library.

9. A method for detecting a familial chylomicronemia syndrome-related gene mutation, which comprises a step of using the capture probe according to any one of claims 1 to 3 or the detection kit according to claim 5 or 6 to capture a target gene fragment containing a familial chylomicronemia syndrome-related gene mutation, and a step of detecting the captured fragment.

10. The method for detecting a familial chylomicronemia syndrome-related gene mutation according to claim 9, wherein the captured fragment is detected by using a sequencing method.
